(19) Europäisches Patentamt
European Patent Office
Office européen des brevets

(11) Publication number: **0 040 082 B1**

(12) # EUROPEAN PATENT SPECIFICATION

(45) Date of publication of patent specification: **25.04.84**

(21) Application number: **81302079.9**

(22) Date of filing: **11.05.81**

(51) Int. Cl.³: **A 01 N 43/40, C 07 D 213/80 //C07D309/38**

(54) **Novel substituted oxonicotinates, their use as plant growth regulators and plant growth regulating compositions containing them.**

(30) Priority: **12.05.80 US 148079**

(43) Date of publication of application: **18.11.81 Bulletin 81/46**

(45) Publication of the grant of the patent: **25.04.84 Bulletin 84/17**

(84) Designated Contracting States: **AT BE CH DE FR GB IT LI LU NL SE**

(56) References cited:
**EP - A - 0 003 144**
**GB - A - 2 029 403**
**US - A - 4 028 084**
**US - A - 4 115 101**

**The file contains technical information submitted after the application was filed and not included in this specification**

(73) Proprietor: **Rohm and Haas Company
Independence Mall West
Philadelphia, Pennsylvania 19105 (US)**

(72) Inventor: **Carlson, Glenn Richard
305 Britt Road
North Wales Pennsylvania, 19454 (US)**

(74) Representative: **Wood, Peter Worsley Spencer et al,
Rohm and Haas Company Patent Department
Chesterfield House Bloomsbury Way
London WC1A 2TP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Novel substituted oxonicotinates, their use as plant growth regulators and plant growth regulating compositions containing them

This invention concerns novel substituted oxonicotinates and their use as plant growth regulators, particularly as chemical sterilants for producing hybrid seeds of monocotyledonous crop plants, for plant breeding purposes or for the agricultural production of ergot; also concerned are plant growth regulating compositions.

The use of chemical sterilants for the production of new cereal grains is a rapidly expanding technology. Cereal grain such as maize, wheat, rice, rye, barley, millet, sorghum, triticale and various monocotyledonous forage crops are the main areas where research has been undertaken to improve both the productivity and the food value of these crops. The utilization of chemical hybridization agents in this research makes possible the hybridization of cereal grain crops on an economical scale. Patents which pertain to this technology include U.S. Patent Nos. 3,761,240, 3,838,155 and 3,576,814 which disclose *N*-aryl-2-oxonicotinates as male sterilants and plant growth regulators. Also U.S. Patent Nos. 4,115,101, 4,051,142 and German Offen. 2,830,700 disclose the use of N-aryl-4-oxonicotinate and *N*-aryl-6-oxonicotinates as male sterilants.

A. Selva and A. Gennaro describe in *Organic Mass Spectrometry,* Vol. *11*, pp 117—120 (1976) the mass spectrometry data for the compound 2-phenyl-6-methyl-3-carbethoxy-4-pyridone. No biological activity is disclosed for this compound.

Balogh et al., *J. Het. Chem., 17,* 359 (1980), disclose the synthesis of various 5-substituted (including 5-phenyl) 1-alkyl-4-oxo-1,4-dihydro-3-pyridine carboxylic acid derivatives for antimicronial studies.

R. Johnstone et al., *Aust. J. Chem. 11,* 562 (1958) (*Chem. Abs., 53,* 5310d (1957)) disclose 1-methyl-6-phenyl-4-pyridone-3-carboxylic acid as a decarboxylation product of a quinolone compound.

T. Kametani et al., *J. Het. Chem. 14,* 477 (1977) disclose the synthesis of various 1,4-dihydro-4-oxonicotinic acid derivatives, some of which bear a 6-phenyl group, which compounds have antibacterial properties.

Wick et al., Ger. Offen. 2,901,868 (*Chem. Abs., 91,* 211273h (1979) disclose 4-pyridone-3-carboxylic acid derivatives, for example, the 6-phenyl derivative thereof, which possess bactericidal and central nervous system stimulant properties.

Adachi, *Chem-Pharm. Bull., 17* (11), 2209 (1969), discloses ring conversion reactions of isoxazolium salts, two of the products of which are ethyl 6-phenyl-1,2,5-trimethyl-4-pyridone-3-carboxylate and 6-phenyl-1,2,5-trimethyl-4-pyridone-3-carboxylic acid. No biological activity is disclosed.

Kigasawa et al., Japan Kokai 78 65,882 (*Chem. Abs., 89,* 129415f (1978) disclose several 1,4-dihydro-4-oxonicotinic acids having antibacterial activity. Among the compounds disclosed are 6-phenyl-5-methyl-1-ethyl-1,4-dihydro-4-oxonicotinic acid, 6-methyl-5-phenyl-1-ethyl-1,4-dihydro-4-oxonicotinic acid, and 5,6-diphenyl-1-ethyl-1,4-dihydro-4-oxonicotinic acid.

The present invention provides novel aryl-substituted nicotinates characterized in that they contain a phenyl (or substituted phenyl) group in the 2-position.

More precisely, the new nicotinates are of Formula I below.

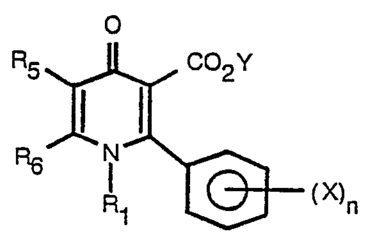

(I)

wherein $R_1$ is $(C_1—C_6)$alkyl or $(C_2—C_6)$alkenyl, each optionally substituted with a group selected from hydroxy, carboxy, carboxy salt, phenyl and phenyl substituted with up to two of the same or different substituents selected from halogen, methyl, ethyl, methoxy, ethoxy, trifluoromethyl, nitro and cyano;

$R_5$ is hydrogen, $(C_1—C_6)$alkyl or halogen;

$R_6$ is hydrogen or $(C_1—C_6)$alkyl;

X is halogen, trihalomethyl, $(C_1—C_6)$alkyl, nitro, cyano or $(C_1—C_6)$alkoxy;

n is 0 or an integer of 1—3; and

Y is hydrogen or $(C_1—C_6)$alkyl.

Also included in the scope of the invention are, where Y = hydrogen, carboxy salts having an agronomically acceptable cation.

The compounds provided by the invention are distinguished from those disclosed in U.S. Patent No. 4,115,101 cited above in that the former compounds exhibit plant growth regulating properties

notwithstanding that the optionally substituted phenyl group is attached to carbon as opposed to nitrogen as is the case with the prior art compounds.

Also included within the scope of the invention are agronomically acceptable acid addition salts of compounds of Formula I. Such salts are possible because the compounds of Formula I possess basic functional groups.

Carboxy salts may be those of lithium, sodium, potassium, an alkaline earth metal, ammonium or substituted ammonium. Acid addition salts may be formed with acids such as hydrochloric, hydrobromic, sulfuric, nitric, perchloric, acetic and oxalic acids.

It will be understood of course that the alkyl and alkenyl groups in the compounds of Formula I may be straight or branched chain. Where in this specification unqualified terms such as propyl or butyl are used the n-alkyl groups are intended.

These compounds are useful as chemical hydridization agents for monocotyldeonous crops and are especially effective in wheat, maize, barley, rice, rye, triticale, sorghum and forage crops.

Those compounds of the invention which are highly potent chemical sterilants which produce a high percentage of male sterility without seriously affecting female fertility of cereal grain and forage crops are especially useful as chemical hybridization agents. As will be seen from the biological data presented later, some of the compounds, particularly at higher application rates, exhibit diminished female fertility along with high male sterility. The latter group of compounds nonetheless have utility for producing new plant hybrids. Although some of the compounds may exhibit some phytotoxicity, the compounds are still useful for the purpose of producing new plant hybrids. In addition to utility as chemical hybridization agents, the compounds of the invention are also useful as chemical sterilants in the agricultural production of ergot. For a description of the use of chemical sterilants for the production of ergot, see French Published Patent Application No. 2400832.

The utility of the compounds of the invention as chemical sterilants was not to be predicted from the prior art listed above.

The most preferred compounds are those of Formula II below, together with the acid addition salts of compounds of Formula II.

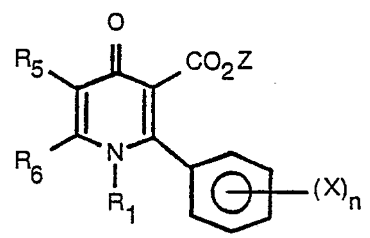

(II)

wherein Z is hydrogen or an alkali metal and $R_1$, $R_5$, $R_6$, X and n are as defined above.

Among the preferred compounds of Formula (II) above are those wherein $R_1$ is $(C_1—C_6)$ alkyl or allyl, $R_5$ is hydrogen, $(C_1—C_3)$ alkyl or bromine, $R_6$ is $(C_1—C_6)$ alkyl, Z is hydrogen or a sodium or potassium cation, X is halogen and n is 0 or the integer 1 or 2.

Among the more preferred compounds of Formula II are those wherein $R_1$ is $(C_1—C_3)$ alkyl, $R_5$ is hydrogen, $R_6$ is $(C_1—C_3)$ alkyl, Z is hydrogen or a sodium or potassium cation, X is chlorine or fluorine and n is 0 or the integer 1 or 2.

Among the most preferred compounds of Formula (II) are those wherein $R_1$ is methyl or ethyl; $R_5$ is hydrogen; $R_6$ is methyl; and Z is a sodium or potassium cation and the agronomically acceptable acid addition salts thereof.

Typical compounds of the invention include:

1,6-dimethyl-2-phenyl-4-oxonicotinic acid
1-ethyl-6-methyl-2-phenyl-4-oxonicotinic acid
1,5,6-trimethyl-2-phenyl-4-oxonicotinic acid
1,6-diethyl-2-phenyl-4-oxonicotinic acid
6-ethyl-1-methyl-2-phenyl-4-oxonicotinic acid
1-methyl-2-phenyl-6-propyl-4-oxonicotinic acid
5-bromo-1,6-dimethyl-2-phenyl-4-oxonicotinic acid
1-allyl-6-methyl-2-phenyl-4-oxonicotinic acid
1,6-dimethyl-2-(4-chlorophenyl)-4-oxonicotinic acid
1-ethyl-6-methyl-2-(4-chlorophenyl)-4-oxonicotinic acid
1-ethyl-5,6-dimethyl-2-(4-chlorophenyl)-4-oxonicotinic acid
1,6-diethyl-2-(4-chlorophenyl)-4-oxonicotinic acid
6-ethyl-1-methyl-2-(4-chlorophenyl)-4-oxonicotinic acid
5-bromo-1-ethyl-6-methyl-2-(4-chlorophenyl)-4-oxonicotinic acid
1-allyl-6-methyl-2-(4-chlorophenyl)-4-oxonicotinic acid

6-methyl-2-(4-chlorophenyl)-1-propyl-4-oxonicotinic acid
1-butyl-6-methyl-2-(4-chlorophenyl)-4-oxonicotinic acid
1,6-dimethyl-2-(3-chlorophenyl)-4-oxonicotinic acid
1-ethyl-6-methyl-2-(3-chlorophenyl)-4-oxonicotinic acid
6-methyl-2-(3-chlorophenyl)-1-propyl-4-oxonicotinic acid
1-ethyl-5,6-dimethyl-2-(3-chlorophenyl)-4-oxonicotinic acid
5-chloro-1-ethyl-6-methyl-2-(3-chlorophenyl)-4-oxonicotinic acid
1-hexyl-6-methyl-2-(3-chlorophenyl)-4-oxonicotinic acid
1-allyl-6-ethyl-2-(3-chlorophenyl)-4-oxonicotinic acid
1,6-diethyl-2-(3-chlorophenyl)-4-oxonicotinic acid
1-ethyl-6-methyl-2-(4-trifluoromethylphenyl)-4-oxonicotinic acid
1-ethyl-6-methyl-2-(4-trifluoromethylphenyl)-4-oxonicotinic acid
1,6-dimethyl-2-(4-fluorophenyl)-4-oxonicotinic acid
1-ethyl-6-methyl-2-(4-fluorophenyl)-4-oxonicotinic acid
1-ethyl-6-methyl-2-(3-fluorophenyl)-4-oxonicotinic acid
1-ethyl-6-methyl-2-(4-bromophenyl)-4-oxonicotinic acid
1,6-dimethyl-2-(3,4-dichlorophenyl)-4-oxonicotinic acid
1-ethyl-6-methyl-2-(3,4-dichlorophenyl)-4-oxonicotinic acid
6-methyl-2-(3,4-dichlorophenyl)-1-propyl-4-oxonicotinic acid
6-methyl-1-pentyl-2-(3,4-dichlorophenyl)-4-oxonicotinic acid
6-ethyl-1-methyl-2-(3,4-dichlorophenyl)-4-oxonicotinic acid
1-ethyl-2-(3,4-dichlorophenyl)-6-propyl-4-oxonicotinic acid
1,5,6-trimethyl-2-(3,4-dichlorophenyl)-4-oxonicotinic acid
5-bromo-1,6-dimethyl-2-(3,4-dichlorophenyl)-4-oxonicotinic acid
1,5-diethyl-6-methyl-2-(3,4-dichlorophenyl)-4-oxonicotinic acid
5-ethyl-1,6-dimethyl-2-(3,4-dichlorophenyl)-4-oxonicotinic acid
1-ethyl-6-methyl-2-(2,4-dichlorophenyl)-4-oxonicotinic acid
1,6-dimethyl-2-(3,5-dichlorophenyl)-4-oxonicotinic acid
1-ethyl-6-methyl-2-(4-methylphenyl)-4-oxonicotinic acid
6-butyl-1-methyl-2-(4-methylphenyl)-4-oxonicotinic acid and the agronomically acceptable salts thereof.

The compounds of the present invention can be prepared by various synthetic routes found in the art.

In particular, the compounds can be prepared by the reaction of a suitably substituted 4-hydroxy-2-pyrone of the formula:

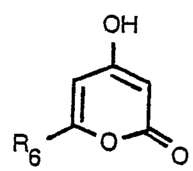

(III)

wherein $R^6$ is as defined above with a benzoyl halide of the formula:

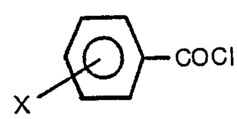

(IV)

wherein X is as defined above in the presence of an acid scavenger such as pyridine and triethylamine, at temperatures from 0 to 10°C to form a 4-benzoyloxy-2-pyrone of the formula:

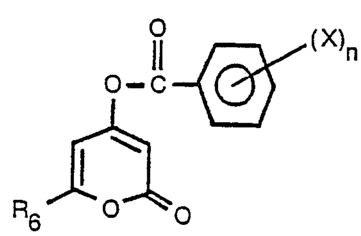

(V)

wherein $R_6$ and X and n are as defined above.

This reaction is discussed in E. Marcus, J. F. Stephen, J. K. Chan, *Journal of Heterocyclic Chemistry,* p. 13, 1966. This benzoate can undergo a Fries-type rearrangement with anhydrous aluminium chloride at elevated temperatures to give the product of the formula:

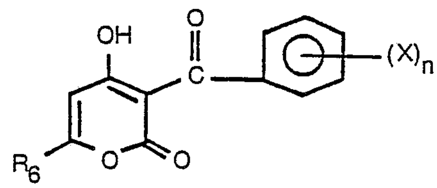

(VI)

as discussed in the E. Marcus, et al. reference, *ibid.* The benzoylpyrone of the formula (VI) above can then be reacted with a suitable alcohol ROH, where R is $(C_1—C_6)$alkyl, in the presence of a similarly substituted trialkylorthoformate $(RO)_3CH$ utilizing an acid catalyst selected from sulfuric, hydrochloric, trifluoroacetic, acetic and hydrobromic at temperatures from 0 to 200°C. to form the 3-carboxy-4-pyrone of the formula:

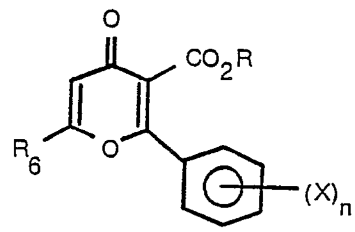

(VII).

The 3-carboxy-4-pyrone esters of formula (VII) can be reacted with any suitably substituted amine of the formula:

$$R_1—NH_2 \qquad (VIII)$$

to yield a 1-alkyl-2-aryl-4-oxonicotinate ester of formula (IX) where $R_5$ is hydrogen.

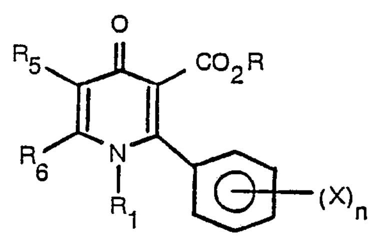

(IX)

This reaction is generally carried out in an inert solvent, such as toluene, xylene, benzene, chloroform, methylene chloride, methanol or ethanol, at room temperature or at a temperature at which the water formed during the reaction can be removed by azeotropic distillation, using 0 to 5% by weight of an acid catalyst such as p-toluenesulfonic acid, hydrochloric acid, sulfuric acid or methanesulfonic acid. The free acid, its salts, and other $(C_1—C_6)$alkyl esters can then be prepared by conventional techniques.

The reaction of 3-carboxy-4-pyrone ester of formula (VII) with excess amine of formula (VIII) in methanol or ethanol at 0—50°C also results in the formation of a 2:1 amine:pyrone adduct of formula (X).

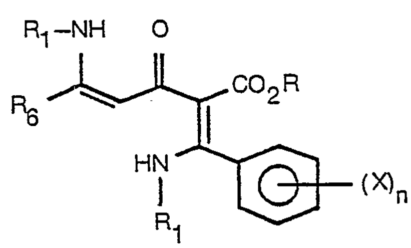

(X)

Compound (X) can be converted to (XI) by hydrolysis with dilute aqueous acids such as hydrochloric, sulfuric, trifluoracetic or methanesulfonic at 0°—50°C. Alternately, 2:1 adduct (X) can be alkylated with an alkyl halide in an inert solvent such as methylene chloride, benzene, tetrahydrofuran and diethyl ether to provide a material of formula (XI) wherein $R_5$ is an alkyl group;

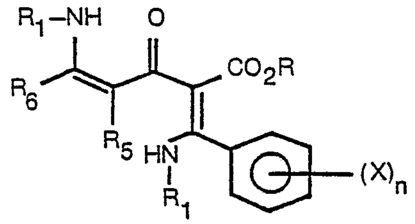

(XI)

This compound can be hydrolyzed in aqueous acids at 0—50°C as described above to yield the corresponding 1,5-dialkyl-2-aryl-4-oxonicotinate ester of formula (IX) where $R_5$=alkyl. The oxonicotinate acid esters produced in the above reactions can be converted to the free acids by hydrolysis with a strong base such as sodium hydroxide or potassium hydroxide followed by neutralization with a strong acid.

Another route to the preparation of the compounds according to Formula II wherein $R_1$ and X are as defined above, $R_5$ is hydrogen and $R_6$ is methyl is depicted in the following reaction sequence:

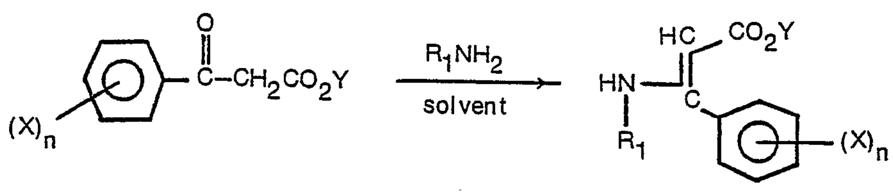


(1)

(XII) (XIII)

wherein Y is a ($C_1$—$C_6$) alkyl group.

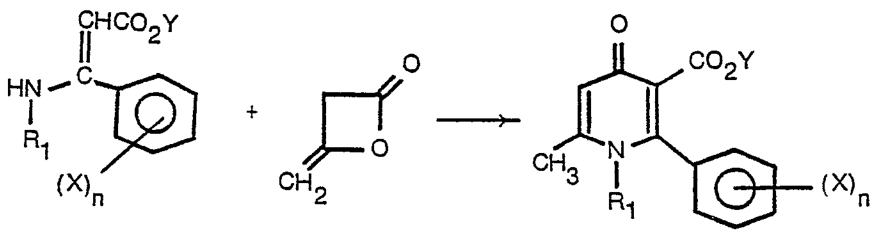

(2)

(XIII) (XIV) (XV)

wherein Y is a ($C_1$—$C_6$) alkyl group.

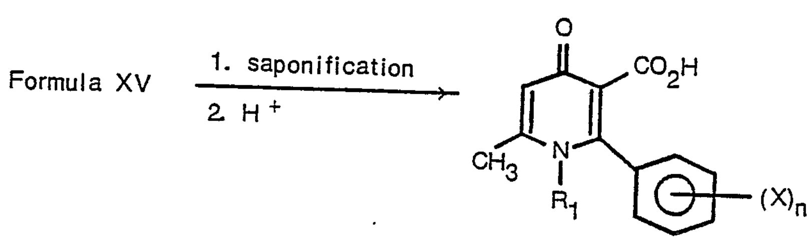


(XVI)

In the above reaction sequences the solvents for (1) can be protic ones, e.g. methanol, ethanol and water and the reaction is run at temperatures from 20° to 100°C. For (2), the inert solvent is selected from ethers, methylene chloride, aromatic hydrocarbons, acetone and acetonitrile and the reaction is run at temperatures from 10° to 150°C. For (3) the saponification reaction is run at 10°—100°C with a strong base, such as sodium or potassium hydroxide, and the alkali metal salt is converted to the free acid *via* mineral acids such as hydrochloride and sulfuric.

Another route to the compounds of Formula I of the invention wherein $R_1$ and $R_6$ are as defined above and $R_5$ is hydrogen or alkyl is shown in scheme (4) below.

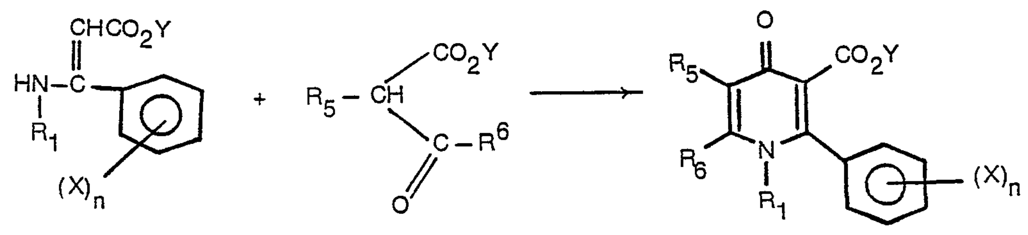

(4)

(XIII) (XVII)

wherein Y is an alkyl group.

In the reaction scheme (4) the reaction can be run either neat or in an inert cosolvent optionally in the presence of an acid catalyst such as toluene sulfonic acid, sulfuric acid and acetic acid at temperatures from 100°C to 300°C.

A route to the 5-halo compounds of Formula II is shown in scheme (5) below.

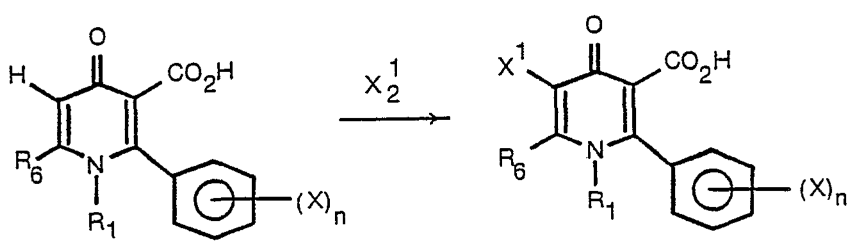


(5)

In this reaction scheme any protic solvent such as water, methanol and ethanol can be utilized as the reaction medium and the reaction can be carried out at temperatures from 10 to 50°C.

The carboxy salts of the oxonicotinic acids of Formula I and II can be prepared by generally known procedures such as dissolving the acids in a protic solvent such as methanol, ethanol and water and treating them with an equivalent amount of a strong base such as sodium or potassium hydroxide, and recovering the salt either by stripping off, the solvent or precipitating the solid out with diethyl ether, hexane and benzene.

Table I below is presented to illustrate the more preferred compounds. In Tables II and III analytical data are presented. In Table II the "calculated" figures are given first and the "found" figures below.

TABLE I

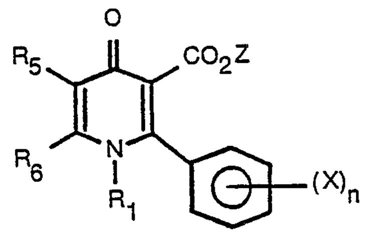

| Compound No. | $X_{(n)}$ | $R_1$ | $R_5$ | $R_6$ | Z |
|---|---|---|---|---|---|
| 1 | H | $CH_3$ | H | $CH_3$ | $CH_3$ |
| 2 | H | $CH_3$ | H | $CH_3$ | $CH_2CH_3$ |
| 3* | H | $CH_3$ | H | $CH_3$ | $CH_2CH_3$ |
| 4 | H | $CH_3$ | H | $CH_3$ | H |
| 4a | H | $CH_3$ | H | $CH_3$ | Na |
| 5 | H | $CH_2CH_3$ | H | $CH_3$ | H |
| 6 | H | n-$C_3H_7$ | H | $CH_3$ | H |
| 6a | H | n-$C_3H_7$ | H | $CH_3$ | Na |
| 7 | H | n-$C_4H_9$ | H | $CH_3$ | H |
| 7a | H | n-$C_4H_9$ | H | $CH_3$ | Na |
| 8 | H | $CH_3$ | H | $CH_2CH_3$ | H |
| 8a | H | $CH_3$ | H | $CH_2CH_3$ | Na |
| 9 | H | $CH_3$ | H | n-$C_3H_7$ | H |
| 9a | H | $CH_3$ | H | n-$C_3H_7$ | Na |
| 10 | H | $CH_2CH_3$ | H | $CH_2CH_3$ | H |
| 10a | H | $CH_2CH_3$ | H | $CH_2CH_3$ | Na |
| 11 | H | $CH_3$ | $CH_3$ | $CH_3$ | H |
| 11a | H | $CH_3$ | $CH_3$ | $CH_3$ | Na |
| 12 | 4-Cl | $CH_3$ | H | $CH_3$ | H |
| 12a | 4-Cl | $CH_3$ | H | $CH_3$ | Na |
| 13 | 4-Cl | $CH_2CH_3$ | H | $CH_3$ | $CH_3$ |
| 14 | 4-Cl | $CH_2CH_3$ | H | $CH_3$ | $CH_2CH_3$ |
| 15 | 4-Cl | $CH_2CH_3$ | H | $CH_3$ | H |
| 15a | 4-Cl | $CH_2CH_3$ | H | $CH_3$ | Na |
| 16 | 4-Cl | $CH_2CH_3$ | $CH_3$ | $CH_3$ | H |
| 16a | 4-Cl | $CH_2CH_3$ | $CH_3$ | $CH_3$ | Na |

TABLE I (cont.)

| Compound No. | $X_{(n)}$ | $R_1$ | $R_5$ | $R_6$ | Z |
|---|---|---|---|---|---|
| 17 | 4-Cl | $n\text{-}C_3H_7$ | H | $CH_3$ | H |
| 17a | 4-Cl | $n\text{-}C_3H_7$ | H | $CH_3$ | Na |
| 18 | 4-Cl | $CH_2CH_2OH$ | H | $CH_3$ | H |
| 18a | 4-Cl | $CH_2CH_2OH$ | H | $CH_3$ | Na |
| 19 | 4-Cl | $CH_2CO_2H$ | H | $CH_3$ | $CH_3$ |
| 19a | 4-Cl | $CH_2CO_2Na$ | H | $CH_3$ | $CH_3$ |
| 20 | 4-Cl | $CH_2CO_2H$ | H | $CH_3$ | H |
| 20a | 4-Cl | $CH_2CO_2Na$ | H | $CH_3$ | Na |
| 21 | 4-Cl | $CH_2CH{=}CH_2$ | H | $CH_3$ | H |
| 21a | 4-Cl | $CH_2CH{=}CH_2$ | H | $CH_3$ | Na |
| 22 | 4-Cl | $n\text{-}C_6H_{13}$ | H | $CH_3$ | H |
| 22a | 4-Cl | $n\text{-}C_6H_{13}$ | H | $CH_3$ | Na |
| 23 | 4-Cl | $CH_2CH_2Ph$ | H | $CH_3$ | H |
| 23a | 4-Cl | $CH_2CH_2Ph$ | H | $CH_3$ | Na |
| 24 | $4\text{-}CH_3$ | $CH_3$ | H | $CH_3$ | H |
| 24a | $4\text{-}CH_3$ | $CH_3$ | H | $CH_3$ | Na |
| 25 | $4\text{-}CH_3$ | $CH_2CH_3$ | H | $CH_3$ | H |
| 25a | $4\text{-}CH_3$ | $CH_2CH_3$ | H | $CH_3$ | Na |
| 26 | 4-F | $CH_3$ | H | $CH_3$ | H |
| 26a | 4-F | $CH_3$ | H | $CH_3$ | Na |
| 27 | 4-F | $CH_2CH_3$ | H | $CH_3$ | H |
| 27a | 4-F | $CH_2CH_3$ | H | $CH_3$ | Na |
| 28 | 4-F | $n\text{-}C_3H_7$ | H | $CH_3$ | H |
| 28a | 4-F | $n\text{-}C_3H_7$ | H | $CH_3$ | Na |
| 29 | 3-Cl | $CH_3$ | H | $CH_3$ | H |
| 29a | 3-Cl | $CH_3$ | H | $CH_3$ | Na |
| 30 | 3-Cl | $CH_2CH_3$ | H | $CH_3$ | $CH_3$ |
| 31 | 3-Cl | $CH_2CH_3$ | H | $CH_3$ | H |
| 31a | 3-Cl | $CH_2CH_3$ | H | $CH_3$ | Na |
| 32 | 3-Cl | $n\text{-}C_3H_7$ | H | $CH_3$ | H |
| 32a | 3-Cl | $n\text{-}C_3H_7$ | H | $CH_3$ | Na |

TABLE I (continued)

| Compound No. | $X_{(n)}$ | $R_1$ | $R_5$ | $R_6$ | Z |
|---|---|---|---|---|---|
| 33 | 3-Cl | n-$C_4H_9$ | H | $CH_3$ | H |
| 33a | 3-Cl | n-$C_4H_9$ | H | $CH_3$ | Na |
| 34 | 3-Cl | $CH_2Ph$ | H | $CH_3$ | H |
| 34a | 3-Cl | $CH_2Ph$ | H | $CH_3$ | Na |
| 35 | 3-$CH_3$ | $CH_2CH_3$ | H | $CH_3$ | H |
| 35a | 3-$CH_3$ | $CH_2CH_3$ | H | $CH_3$ | Na |
| 36 | 3-F | $CH_3$ | H | $CH_3$ | H |
| 36a | 3-F | $CH_3$ | H | $CH_3$ | Na |
| 37 | 3-F | $CH_2CH_3$ | H | $CH_3$ | H |
| 37a | 3-F | $CH_2CH_3$ | H | $CH_3$ | Na |
| 38 | 2-Cl | $CH_3$ | H | $CH_3$ | H |
| 38a | 2-Cl | $CH_3$ | H | $CH_3$ | Na |
| 39 | 2-Cl | $CH_3$ | H | $CH_3$ | H |
| 39a | 2-Cl | $CH_2CH_3$ | H | $CH_3$ | Na |
| 40 | 3,4-diCl | $CH_3$ | H | $CH_3$ | H |
| 40a | 3,4-diCl | $CH_3$ | H | $CH_3$ | Na |
| 41 | 3,4-diCl | $CH_2CH_3$ | H | $CH_3$ | H |
| 41a | 3,4-diCl | $CH_2CH_3$ | H | $CH_3$ | Na |
| 42 | 3,4-diCl | n-$C_3H_7$ | H | $CH_3$ | H |
| 42a | 3,4-diCl | n-$C_3H_7$ | H | $CH_3$ | Na |
| 43 | 2,4-diCl | $CH_3$ | H | $CH_3$ | H |
| 43a | 2,4-diCl | $CH_3$ | H | $CH_3$ | Na |
| 44 | 2,4-diCl | $CH_2CH_3$ | H | $CH_3$ | H |
| 44a | 2,4-diCl | $CH_2CH_3$ | H | $CH_3$ | Na |
| 45 | H | $CH_3$ | Br | $CH_3$ | H |
| 45a | H | $CH_3$ | Br | $CH_3$ | Na |

* Compound No. 3 was in the form the HCl acid addition salt.

TABLE II

| Compound No. | mp(°C) | % C | % H | % N | % X |
|---|---|---|---|---|---|
| 1 | 226—8 | 70.02<br>70.35 | 5.88<br>6.01 | 5.45<br>3.60 | —<br>— |
| 2 | 244—8 | 70.83<br>70.92 | 6.32<br>6.17 | 5.16<br>5.66 | —<br>— |
| 3 | 229—32 | 62.44<br>62.00 | 5.89<br>5.67 | 4.55<br>4.78 | —<br>— |
| 4 | 246—8 | 69.12<br>69.05 | 5.39<br>5.66 | 5.76<br>5.80 | —<br>— |
| 4a | a | — | — | — | — |
| 5 | 252—4 | 70.02<br>69.63 | 5.88<br>5.76 | 5.45<br>5.64 | —<br>— |
| 5a | a | — | — | — | — |
| 6 | 210—2 | 70.83<br>70.67 | 6.32<br>5.93 | 5.16<br>5.33 | —<br>— |
| 6a | a | — | — | — | — |
| 7 | 165—7 | 71.56<br>73.87 | 6.71<br>6.80 | 4.91<br>5.30 | —<br>— |
| 7a | a | — | — | — | — |
| 8 | 244—5 | 70.02<br>70.15 | 5.88<br>5.65 | 5.44<br>6.07 | —<br>— |
| 8a | a | — | — | — | — |
| 9 | 126—8 | 70.83<br>70.61 | 6.32<br>6.39 | 5.16<br>5.36 | —<br>— |
| 9a | a | — | — | — | — |
| 10 | 187—8 | 70.83<br>70.87 | 6.32<br>6.29 | 5.16<br>5.66 | —<br>— |
| 10a | a | — | — | — | — |
| 11 | 210—4 | 70.02<br>69.92 | 5.88<br>5.96 | 5.44<br>5.96 | —<br>— |
| 11a | a | — | — | — | — |
| 12 | 242—4 | 60.55<br>60.17 | 4.36<br>4.36 | 5.04<br>5.53 | 12.77<br>13.07 |
| 12a | a | — | — | — | — |
| 13 | 217—9 | 62.85<br>62.98 | 5.28<br>5.49 | 4.58<br>4.55 | 11.60<br>11.45 |
| 14 | — | — | — | — | — |

TABLE II (continued)

| Compound No. | mp (°C) | % C | % H | % N | % X |
|---|---|---|---|---|---|
| 15 | 235—7 | 61.75<br>60.50 | 4.84<br>4.75 | 4.80<br>5.75 | 12.16<br>13.66 |
| 15a | a | — | — | — | — |
| 16 | 240—2 | 62.85<br>62.86 | 5.28<br>5.37 | 4.58<br>4.71 | 11.60<br>11.48 |
| 16a | a | — | — | — | — |
| 17 | 213—6 | 62.85<br>62.76 | 5.27<br>5.25 | 4.58<br>5.05 | 11.60<br>11.50 |
| 17a | a | — | — | — | — |
| 18 | 217—20 | 58.54<br>58.31 | 4.59<br>4.52 | 4.55<br>4.75 | 11.52<br>11.91 |
| 18a | a | — | — | — | — |
| 19 | 224 | 57.24<br>57.21 | 4.20<br>4.20 | 4.17<br>4.66 | 10.57<br>10.30 |
| 19a | a | — | — | — | — |
| 20<br>(dihydrate) | 158—62 | 50.36<br>50.38 | 4.51<br>3.93 | 3.92<br>3.89 | 9.91<br>9.85 |
| 20a | a | — | — | — | — |
| 21 | 219—21 | 63.26<br>63.30 | 4.65<br>4.59 | 4.61<br>5.09 | 11.67<br>11.75 |
| 21a | a | — | — | — | — |
| 22 | 178—9 | 65.60<br>65.84 | 6.38<br>6.66 | 4.03<br>4.29 | 10.19<br>10.22 |
| 22a | a | — | — | — | — |
| 23 | 265 | 68.57<br>68.48 | 4.93<br>4.95 | 3.81<br>4.12 | 9.64<br>9.44 |
| 23a | a | — | — | — | — |
| 24 | 250 | 70.02<br>69.92 | 5.88<br>6.03 | 5.44<br>5.46 | —<br>— |
| 24a | a | — | — | — | — |
| 25 | 248—9 | 70.83<br>70.64 | 6.31<br>6.39 | 5.16<br>5.60 | —<br>— |
| 25a | a | — | — | — | — |
| 25 | 263—5 | 64.36<br>64.60 | 4.63<br>4.63 | 5.36<br>5.53 | 7.27<br>7.10 |
| 26a | a | — | — | — | — |

TABLE II (continued)

| Compound No. | mp (°C) | % C | % H | % N | % X |
|---|---|---|---|---|---|
| 27 | 258—60 | 65.44<br>65.60 | 5.13<br>5.26 | 5.09<br>5.24 | 6.90<br>6.80 |
| 27a | a | — | — | — | — |
| 28 | 225—6 | 66.42<br>66.60 | 5.58<br>5.74 | 4.84<br>5.11 | 6.57<br>6.47 |
| 28a | a | — | — | — | — |
| 29 | 248—50 | 60.55<br>60.68 | 4.36<br>4.47 | 5.04<br>5.41 | 12.77<br>12.77 |
| 29a | a | — | — | — | — |
| 30 | 173—5 | 62.85<br>62.88 | 5.28<br>5.54 | 4.58<br>5.06 | 11.60<br>11.80 |
| 31 | 241—4 | 61.75<br>62.01 | 4.84<br>4.83 | 4.80<br>4.97 | 12.16<br>12.25 |
| 32 | a | — | — | — | — |
| 32a | 164—9 | 62.85<br>60.83 | 5.28<br>5.40 | 4.58<br>4.34 | 11.60<br>14.09 |
| 33 | a | — | — | — | — |
| 33a | 174—6 | 63.85<br>63.65 | 5.67<br>5.86 | 4.38<br>4.41 | 11.09<br>11.19 |
| 34 | a | — | — | — | — |
| 34a | 235—5 | 67.89<br>68.14 | 4.56<br>4.54 | 3.96<br>4.06 | 10.02<br>10.18 |
|  | a | — | — | — | — |
| 35 | 183 | 70.83<br>70.29 | 6.31<br>6.38 | 5.16<br>5.24 | —<br>— |
| 35a | a | — | — | — | — |
| 36 | 243—4 | 64.36<br>64.34 | 4.63<br>4.96 | 5.36<br>5.40 | 7.27<br>7.07 |
| 36a | a | — | — | — | — |
| 37 | 226—7 | 65.44<br>65.43 | 5.13<br>5.24 | 5.09<br>5.10 | 6.90<br>6.93 |
| 37a | a | — | — | — | — |
| 38 | 221—3 | 60.55<br>60.53 | 4.36<br>4.30 | 5.04<br>4.95 | 12.77<br>17.62 |
| 39 | 212—5 | 61.75<br>61.94 | 4.84<br>4.85 | 4.80<br>4.93 | 12.16<br>12.06 |
| 39a | a | — | — | — | — |

TABLE II (continued)

| Compound No. | mp (°C) | % C | % H | % N | % X |
|---|---|---|---|---|---|
| 40 | 246—9 | 53.87<br>53.40 | 3.55<br>3.55 | 4.49<br>4.42 | 22.72<br>23.10 |
| 40a | a | — | — | — | — |
| 41 | 238—9 | 55.23<br>54.68 | 4.02<br>3.90 | 4.30<br>4.06 | 21.74<br>23.06 |
| 41a | a | — | — | — | — |
| 42 | 232—3 | 56.48<br>56.69 | 4.44<br>4.47 | 4.19<br>4.29 | 20.84<br>20.49 |
| 42a | a | — | — | — | — |
| 43 | 232—4 | 53.87<br>53.96 | 3.55<br>3.57 | 4.49<br>4.88 | 27.72<br>22.69 |
| 43a | a | — | — | — | — |
| 44 | 195 | 55.23<br>55.82 | 4.02<br>4.02 | 4.30<br>4.72 | 21.72<br>21.73 |
| 44a | a | — | — | — | — |
| 45 | 252—3 | 52.19<br>51.72 | 3.76<br>3.67 | 4.35<br>4.26 | 24.81<br>24.78 |
| 45a | a | — | — | — | — |

a) no melting point taken. Too glassy.

TABLE III NMR DATA

| Compound | Solvent | NMR-CHEMICAL SHIFT* |
|---|---|---|
| 1 | $CDCl_3$ | 3H at 2.4 ppm(s); 3H at 3.3 ppm(s); 3H at 3.45 (s); 1H at 6.4 ppm(s); 5H at 7.5 ppm(m) |
| 4 | $CF_3CO_2H$ | 3H at 2.9 ppm(s); 3H at 3.8 ppm(s); 6H at 7.6 ppm(m) |
| 5 | $CF_3CO_2H$ | 3H at 1.4 ppm(t); 3H at 2.9 ppm(s); 2H at 4.5 ppm(q); 6H at 7.7 ppm(m) |
| 7 | $CDCl_3$ | 7H at 0.7—17 ppm(m) 3H at 2.5 ppm(s); 2H at 3.7 ppm(m); 1H at 6.8 ppm(s); 5H at 7.5 ppm(m) |
| 12 | $CDCl_3$+DMSO-$d_6$ | 3H at 2.5 ppm(s); 1H at 3.3 ppm(s); 1H at 6.8 ppm(s); 4H at 7.5 ppm(q) |
| 13 | $CDCl_3$ | 3H at 1.1 ppm(t); 3H at 2.4 ppm(s); 3H at 3.5 ppm(s); 2H at 3.5 ppm(s); 2H at 3.8 ppm(q); 1H at 6.5 ppm(s) 4H at 7.5 ppm(q) |
| 14 | $CDCl_3$ | 3H at 1.2 ppm(t); 3H at 2.75 ppm(s); 2H at 4.2 ppm(q); 1H at 6.9 ppm(s); 4H at 7.9 ppm(m) |
| 15a | $D_2O$ | 3H at 0.8 ppm(t); 3H at 2.3 ppm(s); 2H at 3.7 ppm(q); 1H at 6.6 ppm(s), 4H at 7.6 ppm(s) |

TABLE III, NMR DATA (continued)

| Compound | Solvent | NMR-CHEMICAL SHIFT* |
|---|---|---|
| 16 | DMSO-$d_6$ | 3H at 1.1 ppm(t); 3H at 2.2 ppm(s); 3H at 2.6 ppm(s); 2H at 3.9 ppm(q); 4H at 7.6 ppm(q) |
| 18 | DMSO-$d_6$ | 3H at 2.6 ppm(s); 2H at 3.4 ppm(m); 2H at 3.9 ppm(m); 1H at 6.9 ppm(s); 4H at 7.4 ppm(q) |
| 19 | DMSO-$d_6$ | 3H at 2.3 ppm(s); 3H at 3.4 ppm(s); 2H at 4.5 ppm(s); 1H at 6.4 ppm(s); 4H at 7.6 ppm(q) |
| 24 | $CDCl_3$+DMSO-$d_6$ | 3H at 2.4 ppm(s); 3H at 2.5 ppm(s); 3H at 3.3 ppm(s); 1H at 6.8 ppm(s); 4H at 7.3 ppm(q) |
| 26 | $CF_3CO_2H$ | 3H at 2.8 ppm(s); 3H at 3.8 ppm(s); SH at 7.5 ppm(m) |
| 30 | $CDCl_3$ | 3H at 1.1 ppm(t); 3H at 2.4 ppm(s); 3H at 3.5 ppm(s); 2H at 3.8 ppm(q); 1H at 6.4 ppm(s); 4H at 7.5 ppm(m) |
| 32 | $CDCl_3$ | 3H at 0.7 ppm(t); 2H at 1.5 ppm(m); 3H at 2.6 ppm(s); 2H at 3.6 ppm(q); 1H at 6.8 ppm(s); 4H at 7.5 ppm(m) |
| 33 | $CDCl_3$ | 7H at 0.7—1.7 ppm(m); 3H at 2.5 ppm(s); 2H at 3.7 ppm(m); 1H at 6.8 ppm(s); 4H at 7.4 ppm(m) |
| 35 | $CDCl_3$ | 3H at 1.1 ppm(t); 3H at 2.4 ppm(S); 3H at 2.6 ppm(s); 2H at 3.9 ppm(q); 1H at 6.8 ppm(s); 4H at 7.4 ppm(m) |
| 37 | DMSO-$d_6$ | 3H ar 1.1 ppm(t); 3H at 2.6 ppm(s); 2H at 3.8 ppm(q); 1H at 6.9 ppm(s); 4H at 7.5 ppm(m) |
| 43 | $CF_3CO_2H$ | 3H at 2.9 ppm(s); 3H at 3.9 ppm(s); 4H at 7.7 ppm(m) |
| 44 | $CDCl_3$ | 3H at 1.2 ppm(t); 3H at 2.6 ppm(s); 2H at 3.8 ppm(q); 1H at 6.8 ppm(s); 3H at 7.5 ppm(m) |

* s = singlet
t = triplet
q = quartet
m = multiplet

The following Examples 1—14 are presented to illustrate how the compounds listed in Table I may be prepared. In all of these examples, and in the examples and preparations appearing later, temperatures are in degrees Celsius and percentages are on a weight basis unless otherwise indicated; also where after "mp" there is a parenthetical reference to a solvent, this refers to the recrystallization solvent used.

Example 1 (Compound No. 1, Table I)

*Part a*

A flask fitted with a reflux condenser and calcium chloride drying tube is charged with 70 ml dry methanol, 7.3 gms of 96% sulfuric acid and 10.15 gms of trimethyl orthoformate. 3-Benzoyl-4-hydroxy-6-methyl-2-pyrone (22 gms) is then added in small portions and the resulting reaction mixture is refluxed for 24 hours. The mixture is cooled and poured into water. Extraction with methylene chloride yields 17.7 gms of crude 3-methoxycarbonyl-6-methyl-2-phenyl-4-pyrone. mp (methylene chloride/ether) = 101—2.5.

*Part b*

3.8 gms of 3-methoxycarbonyl-6-methyl-2-phenyl-4-pyrone, 33.3 ml of methanol, 33.3 mls of 40% aqueous methylamine and 2 mls of glacial acetic acid are mixed in a stoppered flask at room temperature and stored for 18 hours. The reaction mixture is then poured into approximately 100 ml of

water and the pH is adjusted to 5 (addition of dilute HCl). Extraction with methylene chloride (3 x 50 mls) and evaporation of the organic solvent yields 3.0 gms of methyl 1,6-dimethyl-2-phenyl-4-oxonicotinate as a crystalline solid. mp (methylene chloride/ether) = 226—8°.

Example 2 (Compound 2, Table I)

10.25 gms of ethyl beta-methylaminocinnamate, 9.4 gms of technical grade diketene and 25 mls dry methylene chloride are mixed in a flask fitted with a calcium chloride drying tube. The reaction mixture is stored at room temperature for 117 hours. Approximately 75 ml of dry ether is added and the resulting crystalline solid is filtered to yield 8.5 gms of ethyl 1,6-dimethyl-2-phenyl-4-oxonicotinate. mp (toluene) = 244—8°.

Example 3 (Compound 4, Table I)

6.0 gms of crude methyl 1,6-dimethyl-2-phenyl-4-oxonicotinate is suspended in 66 gms of 5% aqueous sodium hydroxide solution. The reaction mixture is heated on a steambath (85°) for two hours. The resulting homogeneous solution is cooled and acidified with dilute HCl. The resulting solid is collected by filtration yielding 4.9 gms of 1,6-dimethyl-2-phenyl-4-oxonicotinic acid. mp (acetonitrile/methylene chloride) = 246—9° (dec.).

Example 3a (Compound 4a, Table I)

1.69 gms of 1,6-dimethyl-2-phenyl-4-oxonicotinic acid is suspended in approximately 50 mls of methanol. NaOH pellets (0.305 gms) are added with stirring. After both the acid and the sodium hydroxide dissolve the solution is evaporated to dryness *in vacuo*. Sodium 1,6-dimethyl-2-phenyl-4-oxonicotinate is then isolated as a glassy, somewhat hygroscopic solid. Yield = 1.6 gms.

Example 4 (Compound 8, Table I)

*Part a*

12 gms of ethyl beta-methylaminocinnamate, 18 gms of ethyl propionylacetate and 100 mgs of toluenesulfonic acid monohydrate are added to a 50 ml 3-neck flask fitted with a nitrogen inlet, magnetic stirring bar, thermometer and a short-path distillation head (with receiver). The reaction mixture is heated to 170—5° under a slow stream of dry nitrogen. Ethanol and water are collected in the distillation receiver. After four hours the reaction mixture is cooled and poured into a large volume of ether (150 mls). A pink solid forms which is collected by filtration to yield 4.1 gms of ethyl 6-ethyl-1-methyl-2-phenyl-4-oxonicotinate. mp (ethyl acetate) = 210—2°.

*Part b:*

2.2 gms of ethyl 6-ethyl-1-methyl-2-phenyl-4-oxonicotinate is suspended in 35 ml of 5% aqueous sodium hydroxide solution. The resulting suspension is heated on a steambath for 4 hours, cooled and acidified with dilute HCl to yield 2.0 gms of 6-ethyl-1-methyl-2-phenyl-4-oxonicotinic acid. mp ($CH_3CN$) = 244—5°.

Example 5 (Compound 11, Table I)

*Part a:*

12.0 gms of ethyl beta-methylaminocinnamate, 18 gms of ethyl 2-methylacetoacetate and 100 mgs p-toluenesulfonic acid monohydrate is mixed in a 50 ml 3-neck flask filled with a nitrogen inlet, magnetic stirring bar, thermometer and a short-path distillation head (with receiver). The reaction mixture is heated (170—5°) for seven hours under a gentle stream of nitrogen. Ethanol and water are collected in the distillation receiver. The reaction mixture is cooled and poured into 100 ml of dry ether. Ethyl 1,5,6-trimethyl-2-phenyl-4-oxonicotinate forms as a brownish precipitate.
Yield = 1.03 gms.

*Part b:*

1.03 gms of crude ethyl 1,5,6-trimethyl-2-phenyl-4-oxonicotinate is suspended in 20 mls of warm (85°) 5% aqueous NaOH solution for 4 hours. Acidification with dilute hydrochloric acid yields 0.8 gms of 1,5,6-trimethyl-2-phenyl-4-oxonicotinate mp ($CH_3CN$) = 210—4°.

Example 6 (Compound 13, Table I)

*Part a*:

A 5000 ml 3-neck flask is fitted with a $N_2$ inlet, magnetic stirring bar, thermometer and a Dean-Stark trap with condenser. 2300 mls of dry methanol is added to the flask along with 244 gms of 96% $H_2SO_4$ and 352 gms of trimethyl orthoformate. 173 gms of 3-(4′-chlorobenzoyl)-4-hydroxy-6-methyl-2-pyrone is added and the reaction mixture is gently brought to reflux. The most volatile byproduct of the reaction (methyl formate) is condensed and collected in the Dean-Stark trap. After 28—1/2 hours of reflux the reaction mixture is cooled to room temperature and poured into 6000 mls of brine. The resulting suspension is extracted with methylene chloride (6 x 250 mls). Evaporation of

the solvent yields 153 gms of 3-methoxycarbonyl-6-methyl-2-(4′-chlorophenyl)-4-pyrone. mp (methylene chloride/ether) = 131—2°.

*Part b:*

A 1000 ml flask is fitted with a magnetic stirring bar and a sidearm addition funnel. 500 mls of methanol, 30 mls of glacial acetic acid and 60 gms of 3-methoxycarbonyl-6-methyl-2-(4′-chlorophenyl)-4-pyrone are added. 120 mls of 70% aqueous ethylamine is placed in the addition funnel and added very slowly (4 hr. addition time). 50 mls of water is then added followed by 100 mls of concentrated HCl with cooling. The reaction mixture is allowed to stand undisturbed for 30 minutes. The bulk of the methanol is then removed *in vacuo* leaving 46.1 gms of methyl 1-ethyl-6-methyl-2-(4′-chlorophenyl)-4-oxonicotinate. mp (methylene chloride/ether) = 217.9°.

Example 7 (Compound 15, Table I)

46.1 gms of crude methyl 1-ethyl-6-methyl-2-(4′-chlorophenyl)-4-oxonicotinate is suspended in 600 mls of 5% aqueous NaOH solution and warmed to 80—85°. After $1\frac{1}{2}$ hours at this temperature the reaction mixture is cooled and acidified with dilute HCl. The resulting solid precipitate is collected by filtration, yielding 42 gms of 1-ethyl-6-methyl-2-(4′-chlorophenyl)-4-oxonicotinic acid, mp (methylene chloride/ether) = 235—7°.

Example 8 (Compound 14, Table I)

4.64 gms of ethyl beta-ethylamino-4-chlorocinnamate, 4.61 gms of technical grade diketene and 15 mls of methylene chloride are mixed in a flask fitted with a calcium chloride drying tube. The mixture is maintained at room temperature for 137 hours. The methylene chloride is removed *in vacuo* and the residue triturated with ether to yield 3.2 gms of crude ethyl 1-ethyl-6-methyl-2-(4′-chlorophenyl)-4-oxonicotinate. mp 215—17°.

Example 9 (Compound 14, Table I)

6 gms of ethyl beta-ethylamino-4-chlorocinnamate and 12 gms of ethyl acetoacetate is mixed in a 50 ml 3-neck flask fitted with a $N_2$-inlet, magnetic stirring bar, thermometer and a short-path distillation head (with receiver). The reaction mixture is heated (170°) for 10 hours. Ethanol and water is collected in the distillation receiver. The reaction mixture is cooled and triturated with ethyl acetate/ether to yield 2.52 gms of ethyl 1-ethyl-6-methyl-2-(4′-chlorophenyl)-4-oxonicotinate.

Example 10 (Compound 16, Table I)

*Part a:*

69 gms of 3-methoxycarbonyl-6-methyl-2-(4′-chlorophenyl)-4-pyrone, 500 mls of methanol, 90 mls of water and 30 mls of glacial acetic acid are mixed in a 1000 ml flask fitted with a magnetic stirring bar and a sidearm addition funnel. 120 mls of 70% aqueous ethylamine is added over the course of one hour. The reaction mixture is then allowed to stand at room temperature for one hour, neutralized with dilute HCl and extracted with methylene chloride (2 × 250 mls, 2 × 150 mls). The organic extracts are evaporated and the residue triturated with ether. The first crop of crystals is pure methyl 1-ethyl-6-methyl-2-(4′-chlorophenyl)-4-oxonicotinate (33.1 gms). The solid that forms upon further concentration of the mother liquor is pure alpha-[3-ethylamino-2-butenoyl]-beta-ethylamino-4-chlorocinnamate. Yield = 11 gms. mp = 144 (ether).

*Part b*

7 gms of alpha-[3-ethylamino-2-butenoyl]-beta-ethylamino-4-chlorocinnamate, 5.7 gms methyl iodide, and 20 mls of methylene chloride are mixed in a sealed flask, and stored for 22 hours at room temperature. The contents of the flask are then poured into a separatory funnel and washed with water. Evaporation of the organic layer leaves 9.4 gms of oil which is dissolved in 80 mls of tetrahydrofuran. 60 mls of water and 4 mls of trifluoroacetic acid is then added and the mixture is allowed to remain at room temperature for two hours. Subsequent dilution with water and extraction with methylene chloride provides 8.0 gms of crude 4-oxonicotinate ester. This material is then heated in 80 mls of 5% aqueous sodium hydroxide solution (85°, 2 hrs.) and acidified to yield 2.3 gms of 1-ethyl-5,6-dimethyl-2-(4′-chlorophenyl)-4-oxonicotinic acid. mp = 240.2° (acetonitrile).

Example 11 (Compound 19, Table I)

8.7 gms of 3-methoxycarbonyl-6-methyl-2-(4′-chlorophenyl)-4-pyrone is suspended in 100 mls of methanol and 10 mls of water. In a separate flask, 7.01 gms of glycine, 3.12 gms of sodium hydroxide and 50 mls of methanol are mixed and allowed to stir for 30 minutes to form a solution of the sodium salt of glycine. This solution is then added to the pyrone suspension with stirring. The resulting reaction mixture is allowed to stir at room temperature for five hours. 80 mls of water and 10 mls of concentrated hydrochloric acid is added. A precipitate forms within a few minutes. This is collected by filtration to yield 7.0 gms of methyl 1-carboxymethyl-6-methyl-2-(4′-chlorophenyl)-4-oxonicotinate. mp (methanol/water) = 224.

Example 12 (Compound 20, Table I)

4.0 gms of methyl 1-carboxymethyl-6-methyl-2-(4′-chlorophenyl)-4-oxonicotinate is suspended in 40 gms of 5% aqueous sodium hydroxide solution and heated at 80—85° for two hours. The reaction mixture is cooled and acidified to give a white precipitate. This precipitate is collected by filtration to yield 2.9 gms of 1-carboxymethyl-6-methyl-2-(4′-chlorophenyl)-4-oxonicotinic acid. mp ($CH_3CN$) = 158—62.

Example 13 (Compound 21, Table I)

*Part a*

10 gms of 3-methoxycarbonyl-6-methyl-2-(4′-chlorophenyl)-4-pyrone, 1.65 gms glacial acetic acid and 100 mls of methanol are mixed in 500 ml flask fitted with a magnetic stirring bar and sidearm additional funnel. 6.15 gms of allylamine are slowly added (one hour addition time). Three hours later 20 mls of water and 8 mls of concentrate HCl are added. This reaction mixture is allowed to stand at room temperature for one hour, then is poured into 400 mls of water and extracted with methylene chloride (2 × 100 mls). Removal of the solvent yields 10 gms of crude methyl 1-allyl-6-methyl-2-(4′-chlorophenyl)-4-oxonicotinate.

*Part b*

The crude oxonicotinate ester isolated above is suspended in 80 mls of 5% aqueous sodium hydroxide solution and heated at 85° for 2—1/2 hours. Acidification of this reaction mixture with dilute HCl provides 6.7 gms of 1-allyl-6-methyl-2-(4′-chlorophenyl)-4-oxonicotinic acid. mp (acetonitrile) = 219—21°.

Example 14 (Compound 45, Table I)

6.0 gms of 1,6-dimethyl-2-phenyl-4-oxonicotinic acid is suspended in 200 mls of methanol. 1.0 gms of sodium hydroxide is added. As soon as the acid dissolves a solution of 5.52 gms of $Br_2$ in 50 mls of methanol is slowly added with stirring. The pH of the reaction mixture is maintained at 8—9 by the addition of extra sodium hydroxide as required. A precipitate of 3,5-dibromo-1,6-dimethyl-2-phenyl-pyrid-4-one forms. This material is collected by filtration and discarded. Acidification of the clear filtrate yields 3.4 gms of 5-bromo-1,6-dimethyl-2-phenyl-4-oxo-nicotinic acid as a white solid. mp = 252—3° (acetonitrile).

The compounds of the invention are particularly useful as chemical hybridization agents for the production of hybrid seed of cereal crops, such as wheat, barley, corn, rice, sorghum, millets, oats, rye and triticale. When used for this purpose, the compounds are so chosen as to effectively induce a high degree of selective male sterility, that is without also inducing significant female sterility, in the treated plants and without causing significant growth inhibition of the treated plants. As used herein, the term male sterility includes both actual male sterility, as evidenced by a lack of male flower parts or by sterile pollen, and functional male sterility, in which the male flower parts are unable to cause pollination.

The compounds of the invention may also cause other plant growth regulatory responses, such as for example control of flowering, control of fruiting and inhibition of seed formation and other related growth regulatory responses.

When used as plant growth regulators, the compounds of the invention are applied in any amount which will be sufficient to effect the desired plant response. For example, when the compounds of the invention are used as chemical hybridization agents, they are generally applied to the crops to be treated at a rate of 0.035 kg to 22.42 kg per hectare (1/32 to 20 pounds per acre) and preferably 0.14 to 11.21 kg per hectare (1/8 to 10 pounds per acre).

The rate of application will vary depending on the crop being treated, the compound being used for treatment, and related factors.

To obtain hybrid seed, the following procedure is generally employed. The two parents to be crossed are planted in alternate strips. The female parent is treated with a compound of the invention. The male-sterile female parent thus produced will be pollinated by pollen from the other, male-fertile, male parent, and the seed produced by the female parent will be hybrid seed which can then be harvested by conventional means.

A preferred method of applying a compound of the invention as a chemical hybridization agent is by foliar application. When this method is employed, selective male sterility is most effectively induced when the compound is applied between another initiation and meiosis. The compounds of the inventions may also be applied as a seed treatment by soaking the seed in a liquid formulation containing the active compound or by coating the seed with the cmpound. In seed treatment applications, the compounds of the invention will generally be applied at a dose of 113.4 g to 4.54 kg per 45 kg (1/4 to 10 pounds per %). The compounds of the invention are also effective when applied to the soil or to the water surface in rice crops.

The compounds of the invention can be used as plant growth regulators either individually or in mixtures. For example, they can be used in combination with other plant growth regulators, such as auxins, gibberellins, ethylene-releasing agents such as ethephon, pyridones, pyridazinones, cytokinins, maleic hydrazide, succinic acid 2,2-dimethylhydrazide, chlorine and its salts (2-chloroethyl)

trimethylammonium chloride, triiodobenzoic acid, tributyl-2, 4-dichlorobenzylphosphonium chloride, polymeric N-vinyl-2-oxazolidinones, tri)dimethylaminoethyl) phosphate and its salts, and N-dimethylamino-1,2,3,6-tetrahydrophthalamic acid and its salts, and under some conditions may be used advantageously with other agricultural chemicals such as herbicides, fungicides, insecticides, and plant bactericides.

A compound of the invention can be applied to the growth medium or to plants to be treated either by itself or, as is generally done, as a component in a growth regulant composition or formulation which also comprises an agronomically acceptable carrier. By "agronomically acceptable carrier" is meant any substance which can be used to dissolve, disperse, or diffuse a compound in the composition without impairing the effectiveness of the compound and which by itself has no significant detrimental effect on the soil, equipment, crops or agronomic environment. Mixtures of the compounds of the invention may also be used in any of these formulations. The compositions of the invention can be either solid or liquid formulations or solutions. For example, the compounds can be formulated as wettable powders, emulsifiable concentrates, dusts, granular formulations, aerosols, or flowable emulsion concentrates. In such formulations, the compounds are extended with a liquid or solid carrier and, when desired suitable surfactants are incorporated.

It is usually desirable, particularly in foliar applications, to include adjuvants, such as wetting agents, spreading agents, dispersing agents, stickers and adhesives, in accordance with agricultural practices. Examples of adjuvants which are commonly used in the art can be found in the John W. McCutcheon, Inc. publication "Detergents and Emulsifiers Annual."

The compounds of the invention can be dissolved in any appropriate solvent. Examples of solvents which are useful in the practice of this invention include water, alcohols, ketones, aromatic hydrocarbons, halogenated hydrocarbons, dimethylformamide, dioxane, dimethyl sulfoxide, and the like. Mixtures of these solvents can vary from 2% to 98% by weight with a preferred range being from 20% to 75%.

For the preparation of emulsifiable concentrates, the compound can be dissolved in organic solvents, such as benzene, toluene, xylene, methylated naphthalene, corn oil, pine oil, o-dichlorobenzene, isophorone, cyclohexanone and methyl oleate, or in mixtures of these solvents, together with an emulsifying agent or surfactant which permits dispersion in water. Suitable emulsifiers include, for example, the ethylene oxide derivative of alkylphenols or long-chain alcohols, mercaptans, carboxylic acids, and reactive amines and partially esterified polyhydric alcohols, Solvent-soluble sulfates or sulfonates, such as the alkaline earth salts or amine salts of alkyl-benzenesulfonates and the fatty alcohol sodium sulfates, having surface-active properties can be used as emulsifiers either alone or in conjunction with an ethylene oxide reaction product. Flowable emulsion concentrates are formulated similarly to the emulsifiable concentrates and include, in addition to the above components, water and a stabilizing agent such as a water-soluble cellulose derivative or a water-soluble salt of a polyacrylic acid. The concentration of the active ingredient in emulsifiable concentrates of usually 10% to 60% by weight and in flowable emulsion concentrates, this can be as high as about 75%.

Wettable powders suitable for spraying, can be prepared by admixing the compound with a finely divided solid, such as clays, inorganic silicates and carbonates, and silicas and incorporating wetting agents, sticking agents and/or dispersing agents in such mixtures. The concentration of active ingredients in such formulations is usually in the range of 20% to 98% by weight, preferably 40% to 75%. A dispersing agent may generally constitute 0.5% to 3% by weight of the composition, and a wetting agent may generally constitute from 0.1% to 5% by weight of the composition.

Dusts can be prepared by mixing the compounds of the invention with finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing 20% to 80% of the active ingredient are commonly made and are subsequently diluted to 1% to 10% by weight use concentration.

Granular formulations can be prepared by impregnating a solid such as granular Fuller's earth, vermiculite, ground corn cobs, seed hulls, including bran or other grain hulls, or similar material. A solution of one or more of the compounds in a volatile organic solvent can be sprayed or mixed with the granular solid and the solvent then removed by evaporation. The granular material can have any suitable size, with a preferable size range of 16 to 60 mesh. The active compound will usually comprise 2 to 15% by weight of the granular formulations.

Salts of the compounds of the invention can be formulated and applied as aqueous solutions. The salt will typically comprise 0.05 to 50% by weight preferably 0.1% to 10%, of the solution.

These compositions can also be further diluted with water if desired prior to actual application. In some applications, the activity of these compositions can be enhanced by incorporating into the composition an adjuvant such as glycerin, methylethylcellulose, hydroxyethylcellulose, polyoxyethylenesorbitan monooleate, polypropylene glycol, polyacrylic acid, polyethylene sodium malate or polyethylene oxide. Such adjuvants will generally comprise 0.1 to 5% by weight, preferably 0.5 to 2%, of the composition. Such compositions can also optionally include an agronomically acceptable surfactant.

The compounds of the invention can be applied as sprays by methods commonly employed, such as conventional hydraulic sprays, aerial sprays, and dusts. For low volume applications, a solution of the compound is usually used. The dilution and volume of application will usually depend upon such factors as the type of equipment employed, the method of application, the area to be treated and the type and state of development of the crop being treated.

The following is a typical tank mix formulation for the foliar application of sodium 1-ethyl-6-methyl-2-(4-chlorophenyl)-4-oxonicotinate on winter wheat at a dose of 0.28 kg/ha (0.25 lbs/A) active ingredient:

0.1134 kg (0.25 lbs) of oxonicotinate salt
0.568 l (1 pint) of Triton®AG 98
dilute to 189.25 l (50 US gals) with water.

This mixture is then foliar spray-applied to the wheat plants at a rate of 467.5 l/ha (50 US gals/A).

The following data illustrate the chemical growth regulating activity of the compounds of the invention. In Tables IV to XII which follow the symbol "D" indicates a dose of 8.968 kg/ha (8 lb/acre) of active ingredient and the symbols "D/2" etc. mean that the doses were progressively halved so that D/2 = 4.484 kg/ha (4 lb/acre) etc. to D/256 = 0.035 kg/ha (1/32 lb/acre).

Growth Regulating

The following procedures are used to evaluate the activity of the compounds of the invention for inducing male sterility in cereals.

An awned variety (Fielder) and an awnless variety (Mayo-64) of spring wheat are planted at the rate of 6 to 8 seeds per 15.24 cm (6 inch) diameter pot containing a sterile medium of 3 parts soil and 1 part humus. The plants are grown under short-day (9 hour) conditions for the first 4 weeks to obtain good vegetative growth before flower initiation. The plants are then moved to long-day (16 hour) conditions which are provided by high intensity lights in the greenhouse. The plants are fertilized at 2, 4 and 8 weeks after planting with a water-soluble fertilizer (16—25—16 by weight NPK) at the rate of 1.3 ml/l (1 teaspoon/US gal) of water, and are frequently sprayed with Isotox (benzenehexachloride) for aphid control and dusted with sulfur for powdery mildew control.

Test compounds are foliarly applied to the awned female plants when these plants reach the flag leaf emergence stage (stage 8 on Feekes scale — W. Feekes, Vers 17 Tech Tarwe Comm, Groningen, pp. 560—561, 1941). All compounds are applied in a carrier (water) volume of 467.5 l/ha (50 US gal/A) containing the surfactant Triton X-100 at the rate of 567 g/189.25 l (20 oz/50 US gal).

After spike emergence but before anthesis, 4 to 6 spikes per pot are bagged to prevent outcrossing. At the first signs of flower opening, two spikes per pot are cross pollinated, using the approach method, with the awnless male parent. As soon as the seeds become plainly visible, spike length is measured and seeds per spikelet counted in both bagged and crossed spikes. Male sterility can then be calculated as percent inhibition of seed set in bagged spikes of treated plants, and female fertility in crossed spikes can be calculated as percent of control seed set. After maturity the seed on crossed spikes are planted for determination of percent hybridization.

Percent sterility, percent fertility, and percent height inhibition are calculated from the following formulas:

a. % Sterility = $(S_c - S_t/S_c) \times 100$
$S_c$ = seeds/spikelet in bagged spikes of control plants.
$S_t$ = seeds/spikelet in bagged spikes of treated plants.

b. % Fertility = $(F_t/F_c) \times 100$
$F_t$ = seeds/spikelet in approach crossed spikes of treated plants
$F_c$ = seeds/spikelet in unbagged spikes of control plants

c. % Height inhibition = $(H_c - H_t/H_c) \times 100$
$H_c$ = Height of control plants
$H_t$ = Height of treated plants

Table IV summarizes typical results obtained in the evaluation of representative compounds of the invention. A dash in this and the following tables indicates that no determination of value was made.

TABLE IV

Biological Data (Wheat Greenhouse Data)
% Male Sterility

| Compound No. | D | D/2 | D/4 | D/8 | D/16 | D/32 | D/64 |
|---|---|---|---|---|---|---|---|
| 4a | — | 100 | 100 | 77 | 36 | — | — |
| 5a* | 100 | — | 100 | — | 43 | — | 2 |
| 6a | 100 | — | 85 | — | 18 | — | 54 |
| 8a | — | — | 98 | — | 34 | — | 1 |
| 9a | 99 | — | 88 | — | 32 | — | 5 |
| 10a | — | — | 100 | — | 100 | — | 13 |
| 11a | — | 88 | — | 2 | — | 2 | — |
| 12a | — | 100 | 100 | 92 | 55 | 12 | — |
| 15a | — | 100 | 100 | 100 | 96 | 91 | — |
| 16 | — | 100 | 100 | 100 | 96 | 91 | — |
| 16a | 100 | — | 100 | 18 | 0 | — | 0 |
| 17a | — | — | — | 100 | 66 | 100 | 41 |
| 21a | 100 | — | — | — | — | — | — |
| 24a | — | 55 | 49 | 0 | 0 | 0 | — |
| 25a | — | 94 | 7 | 15 | 0 | 2 | — |
| 26a | — | 100 | 100 | 57 | 0 | 2 | — |
| 27a | — | 100 | 100 | 100 | 39 | 7 | 0 |
| 28a | — | 100 | 70 | 1 | 0 | 0 | — |
| 29a | — | 100 | 100 | 100 | 0 | 0 | — |
| 31a | — | 100 | 100 | 100 | 100 | 87 | — |
| 32a | 100 | — | 100 | — | 100 | — | 37 |
| 33a | 100 | — | 79 | — | 50 | — | 44 |
| 35a | 100 | — | 100 | — | 13 | — | 1 |
| 36a | — | — | — | 90 | 57 | — | — |
| 37a | — | — | — | 90 | 65 | 0 | 0 |
| 38a | 100 | — | 100 | — | 45 | — | 0 |
| 39a | 100 | — | 92 | — | 14 | — | 0 |
| 40a | 100 | — | 100 | — | 90 | — | 43 |

* No salt of Compound 5

TABLE IV (continued)

| Compound No. | D | D/2 | D/4 | D/8 | D/16 | D/32 | D/64 |
|---|---|---|---|---|---|---|---|
| 41a | 100 | — | 100 | — | 100 | — | 100 |
| 42a | 100 | — | 100 | — | 80 | — | 4 |
| 43a | 100 | — | 100 | — | 93 | — | 8 |
| 44a | 100 | — | 100 | — | 73 | — | 0 |
| 45a | 100 | — | 84 | — | 0 | — | 0 |

Table V below depicts the male/female selectivity of several of the compounds of Formula I. Doses just sufficient to effect high levels of male sterility maintain useful levels of female sterility. Substantial loss of female fertility occurs only at doses above those required to effect high levels of male sterility.

TABLE V

Male Sterility, Female Fertility, Culm Inhibition, Height Inhibition (Wheat Greenhouse Data)

| Compound No. | Dose | % Male Sterility | % Culm Inhibition | % Spike Inhibition | % Female Fertility |
|---|---|---|---|---|---|
| 12a | D/8 | 100 | 15 | 23 | — |
| | D/16 | 93 | 5 | 9 | 23 |
| | D/32 | 100 | 0 | 0 | 67 |
| | D/64 | 98 | 0 | 11 | 80 |
| | D/128 | 10 | 0 | 0 | — |
| 15a | D/8 | 100 | 50 | 28 | — |
| | D/16 | 100 | 40 | 14 | — |
| | D/32 | 100 | 20 | 14 | — |
| | D/64 | 100 | 25 | 9 | 70 |
| | D/128 | 83 | 0 | 4 | — |
| 42a | D/8 | 100 | 0 | 0 | 78 |
| | D/16 | 100 | 0 | 2 | 80 |
| 42a | D/32 | 70 | 5 | 4 | — |
| | D/64 | 40 | 10 | 5 | — |

Other crops upon which compounds of the present invention have been shown to be effective are maize and barley.

The following procedures are used to evaluate the activity of the compounds of this invention for inducing male sterility in maize.

Four rows of inbred variety B-73 are interplanted with two rows of inbred variety Mo-17. Both lines are planted at a rate of 20000 seeds/0.4047 ha (20,000 seeds/A). The chemicals of this invention are applied to variety B-73 several weeks later when the last leaf of the plant is just beginning to elongate and the tassel is approximately one inch (2.5 cm) long and not yet branched. All treatments

are applied with a hand held pressurized sprayer equipped with a 3-nozzle boom containing D3 disc/45 core Tee Jet cone spray tips. Each nozzle is directed at the row with one placed on either side and over the row. Triton® X-100 surfactant at 0.093% by weight is added as a surfactant. Dose and carrier volume calculations are based on rows 91.44 cm (36 inches) wide.

Pollinating ability of treated plants is measured by crossing their pollen onto silks of untreated plants and recording the amount of seed set.

Table VI below demonstrates the percent male sterility effected by Compound 15a on field maize.

TABLE VI

| Compound No. | D | D/2 | D/4 | D/8 | D/16 | D/32 | D/64 |
|---|---|---|---|---|---|---|---|
| 15a | — | 100 | 100 | 100 | 100 | 100 | — |

The compounds of the present invention are also effective on barley.

Table VII below demonstrates the % male sterility obtained with one of the compounds of the present invention on barley grain (variety Park) under greenhouse conditions.

TABLE VII

| Compound No. | D/32 | D/64 | D/128 | D/256 |
|---|---|---|---|---|
| 15a | 100 | 96 | 95 | 88 |

Table VIII below sets forth field test data which demonstrate further the effect of several compounds of the present invention on the male sterility of field maize (B-73).

TABLE VIII

% Male Sterility of Field Corn (Field Test Data)

| Compound No. | D/128 | D/64 | D/32 | D/16 | D/8 | D/4 | D/2 | D |
|---|---|---|---|---|---|---|---|---|
| 41a | 100 | 100 | 100 | 100 | — | — | — | — |
| 15a | 13 | 67 | 99 | 100 | 100 | — | — | — |
| 15,K salt | 12 | 40 | 100 | 100 | 100 | — | — | — |
| 31a | 42 | 84 | 100 | 100 | — | — | — | — |
| 29a | — | 64 | 34 | 100 | 100 | — | — | — |
| 12a | — | 29 | 59 | 62 | 90 | 100 | 100 | — |
| 17a | — | — | 8 | 30 | 80 | 100 | 100 | — |
| 5a | — | — | 37 | 52 | 98 | 100 | 100 | — |
| 25a | — | — | 70 | 100 | 100 | 100 | 100 | — |
| 4a | — | — | — | — | 12 | 67 | 88 | 100 |

Table IX below sets forth additional field test data which demonstrate further the effect of several compounds of the present invention on the male sterility and female fertility of field maize (B-73).

TABLE IX

% Male Sterility and Female Fertility of Field Maize
(Field Test Data)
% Male Sterility

| Compound No. | D/128 | D/64 | D/32 | D/16 | D/8 | D/4 |
|---|---|---|---|---|---|---|
| 15, K salt | 90 | 100 | 100 | 100 | 100 | 100 |
| 12, K salt | 27 | 58 | 62 | 88 | 86 | 100 |
| 41, K salt | 90 | 100 | 100 | 100 | 100 | — |
| 40, K salt | 16 | 46 | 72 | 100 | 100 | 100 |
| 31a | 82 | 100 | 100 | 100 | 100 | 100 |
| 29a | 52 | 65 | 65 | 59 | 47 | 34 |

% Female Fertility*

| Compound No. | D/128 | D/64 | D/32 | D/16 | D/8 | D/4 |
|---|---|---|---|---|---|---|
| 15, K salt | G—E | G—E | G—E | G | P—F | P |
| 12, K salt | E | G—E | G—E | E | G | F—G |
| 41, K salt | G—E | G | F—G | G | G | — |
| 40, K salt | G—E | G | G | G | G | G |
| 31a | G—E | G | G | F | P | P |
| 29a | G—E | G | G—E | G | G—E | G |

* E=Excellent; G=Good; F=Fair; P=Poor.

Table X below sets forth field test data which demonstrate the effect of several compounds of the present invention on the male sterility and female fertility of sorghum (Inbred No. 6250).

TABLE X

% Male Sterility and % Female Fertility of Sorghum
(Field-Test Data)

| | % Sterility | | | | % Fertility* | | | |
|---|---|---|---|---|---|---|---|---|
| Compound No. | D/64 | D/32 | D/16 | D/8 | D/64 | D/32 | D/16 | D/8 |
| 15, K salt | 97 | 99 | 100 | 100 | E | E | E | F |
| 41, K salt | 100 | 100 | 100 | 100 | P | P | P | P |
| 40, K salt | 83 | 98 | 100 | 100 | E | E | E | G |

* E=Excellent; G=Good; F=Fair; P=Poor.

The Table XI below sets forth additional field test data which demonstrate the effectiveness of several compounds of the present invention on male sterility and female fertility of sorghum (Inbred No. 7078).

TABLE XI

% Male Sterility and % Female Fertility of Sorghum
(Field Test Data)

| Compound No. | % Male Sterility | | | | % Female Fertility* | | | |
|---|---|---|---|---|---|---|---|---|
| | D/64 | D/32 | D/16 | D/8 | D/64 | D/32 | D/16 | D/8 |
| 15, K salt | 35 | 73 | 95 | 100 | E | E | G | F |
| 41, K salt | 100 | 100 | 100 | 100 | E | F | P | P |
| 40, K salt | 9 | 34 | 91 | 100 | E | E | E | F |

* E=Excellent; G=Good; F=Fair; P=Poor.

Table XII below sets forth field test data which demonstrate the effect of several compounds of the present invention on male sterility and female fertility of barley (Variety Henry).

TABLE XII

% Male Sterility and % Female Fertility of Barley
(Field Test Data)

| Compound No. | % Sterility | | | | % Fertility | | | |
|---|---|---|---|---|---|---|---|---|
| | D/64 | D/32 | D/16 | D/8 | D/64 | D/32 | D/16 | D/8 |
| 15, K salt | 32 | 56 | 92 | 99 | 93 | 96 | 75 | 56 |
| 12, K salt | 2 | 4 | 4 | 8 | 95 | 100 | 100 | 96 |
| 41, K salt | 4 | 22 | 48 | 77 | 99 | 97 | 94 | 76 |
| 40, K salt | 3 | 4 | 4 | 43 | 96 | 100 | 100 | 81 |

There should also be mentioned as specific compounds of Formula I and carboxy and acid addition salts thereof all the individual compounds hereinbefore disclosed (together with those additional compounds specified in paragraphs (b) to (f) below) but in which, referring to the symbols used in Formulae I and II.

(a) $—(X)_n$ in the individual compound in question is replaced by different group(s) or atom(s) selected from bromine, fluorine, chlorine, iodine, trichloromethyl, tribromomethyl, trifluoromethyl, triodomethyl, methyl, ethyl, propyl, butyl, pentyl, hexyl (and all possible isomers of the last 4 groups), nitro, cyano, methoxy, ethoxy, propoxy, butoxy, pentoxy, hexyloxy (and all possible isomers of the last 4 groups) and all combinations of the above listed groups, e.g. a combination such as 2-chloro-3-methyl-4-methoxy;

(b) $R_1$ in the individual compound in question, is replaced by a different group selected from methyl, ethyl, propyl, butyl, pentyl, hexyl (and all possible isomers of the last 4 groups), vinyl, propenyl, butenyl, pentenyl, hexenyl (and all possible isomers of the last 4 groups) and each of the alkyl and alkenyl groups listed in the sub-paragraph (b) substituted with hydroxy, carboxy, carboxy salts (e.g. $CO_2Na$, $CO_2K$ and $CO_2Li$), phenyl, phenyl substituted with up to two of the same or different substituents selected from chlorine, bromine, fluorine, iodine, methyl, ethyl, methoxy, ethoxy and trifluoromethyl;

(c) $R_5$ in the individual compound in question is replaced by a different group or atom selected from hydrogen, methyl, ethyl, propyl, butyl, pentyl, hexyl (and all possible isomers of the last 4 groups), chlorine, fluorine, bromine and iodine;

(d) $R_6$ in the individual compound in question is replaced by a different group or atom selected from hydrogen, methyl, ethyl, propyl, butyl, pentyl and hexyl including all possible isomers of the last 4 groups;

(e) Y in the individual compound in question is replaced by a different atom or group selected from hydrogen, methyl, ethyl, propyl, butyl, pentyl and hexyl including all possible isomers of the last 4 groups; and

(f) Y or (as the case may be) Z in the individual compound in question is replaced by a different

atom or group selected from hydrogen, sodium, potassium, lithium, calcium, barium, strontium, magnesium, ammonium and ammonium substituted by one, two, three or four of the same or different ($C_1$—$C_6$) alkyl groups, viz. methyl, ethyl, propyl, butyl, pentyl and hexyl including all possible isomers of the last 4 groups.

Broadly, the compounds of the invention find applications as plant growth regulating agents by a method which involves regulating the growth of a monocotyledonous crop plant by applying to the growing plant, or to seeds thereof, a growth regulating amount of one or more of the compounds in question. The preferred application is that of producing hybrid seed by the use of the compounds as selective male sterilants whereby either hybrid seed of enhanced commercial value is thus directly obtained or hybrid seed is obtained which is of utility in plant breeding programmes. In one application of the compounds as chemical hybridizing agents, hybrid seed of a gramineous plant is produced by applying one or more of the compounds to the seeds of the plant, or to the foliage of the plant prior to meiosis, whereby male sterility is induced without destruction of female fertility. The thus treated plant is then pollinated with pollen from a male parent of a different gramineous plant and which is such as to yield the desired hybrid seeds. The pollinated plant is then allowed to mature until seed formation and mature hybrid seeds are collected. Such a method for the production of hybrid seed may involve charging to a container or mechanical dissemination device a hybridization composition containing one or more compounds of the invention and an agronomically acceptable carrier therefor. The thus charged container or mechanical dissemination device is then used to apply the hybridization composition to the plant or seeds thereof, e.g. by the application of a dust or liquid spray to the seeds or plant foliage or by immersing the seeds in a liquid hybridization composition in the container.

For the agricultural production of ergot by infecting a gramineous plant with ergot spores, allowing the sclerotia to develop and harvesting the mature ergot sclerotia, the method involves applying to the plant prior to infection with the ergot spores at least one compound of the invention in an amount effective to produce significant male sterility in the plant.

**Claims for the Contracting States: BE CH DE FR GB IT LI LU NL SE**

1. A compound of Formula I below, or an agronomically acceptable carboxy salt thereof (where Y in the formula is hydrogen) or an agronomically acceptable acid addition salt thereof,

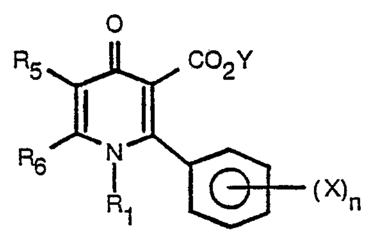

(I)

wherein $R_1$ is ($C_1$—$C_6$)alkyl or ($C_2$—$C_6$)alkenyl, each optionally substituted with a group selected from hydroxy, carboxy, carboxy salt, phenyl and phenyl substituted with up to two of the same or different substituents selected from halogen, methyl, ethyl, methoxy, ethoxy, trifluoromethyl, nitro and cyano;

$R_5$ is hydrogen, ($C_1$—$C_6$)alkyl or halogen;

$R_6$ is hydrogen or ($C_1$—$C_6$)alkyl;

X is halogen, trihalomethyl, ($C_1$—$C_6$)alkyl, nitro, cyano or ($C_1$—$C_6$)alkoxy;

n is 0 or an integer of 1—3; and

Y is hydrogen or ($C_1$—$C_6$)alkyl.

2. A compound as claimed in Claim 1 which is of the formula:

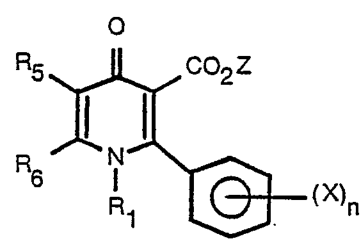

(II)

wherein: $R_1$, $R_5$, $R_6$, X and n are as defined in Claim 1 and Z is hydrogen or an alkali metal and the agronomically acceptable acid addition salts thereof.

3. A compound or acid addition salt as claimed in Claim 2 wherein

$R_1$ is ($C_1$—$C_6$) alkyl or allyl;

$R_5$ is hydrogen, bromine or ($C_1$—$C_3$) alkyl;
$R_6$ is ($C_1$—$C_6$) alkyl;
Z is hydrogen, sodium or potassium;
X is halogen; and
n is 0, 1 or 2.

4. A compound or acid addition salt as claimed in Claim 3, wherein
$R_1$ is ($C_1$—$C_3$) alkyl;
$R_5$ is hydrogen;
$R_6$ is ($C_1$—$C_3$) alkyl;
X is chlorine or fluorine; and
n is 0, 1 or 2.

5. A compound or acid addition salt as claimed in Claim 4 wherein $R_1$ is methyl or ethyl; $R_6$ is methyl; and Z is sodium or potassium.

6. A compound or acid addition salt as claimed in Claim 5 wherein $R_1$ is ethyl, $R_6$ is methyl and $(X)_n$ is hydrogen, 4-chloro, 3-chloro, 3,4-dichloro, or 3-fluoro.

7. A compound or acid addition salt as claimed in Claim 6 wherein $(X)_n$ is 4-chloro and Z is potassium.

8. A compound as claimed in Claim 5, wherein $R_1$ is methyl, $R_6$ is methyl and $(X)_n$ is 3-chloro, 4-chloro, or 3,4-dichloro.

9. A growth regulating composition containing as active ingredient one or more compounds as claimed in any preceding claim and an agronomically acceptable carrier therefor.

10. A composition as claimed in Claim 9, wherein the carrier is water and there is also present a surface active agent.

11. A method of regulating the growth of a monocotyledonous crop plant which comprises applying to the growing plant, or to seeds thereof, a growth regulating amount of one or more compounds as claimed in any one of Claims 1—8.

12. A method as claimed in Claim 11 as applied to the production of hybrid seed by using at least one growth regulating compound which induces male sterility without destroying female fertility.

13. A method as claimed in Claim 12 wherein the plant is maize, barley, wheat, sorghum or a forage crop plant.

**Claims for the Contracting State: AT**

1. A plant growth regulating composition containing, as active ingredient, at least one compound which induces male sterility in monocotyledenous plants and an agronomically acceptable carrier or diluent therefor, characterized in that it contains at least one compound of Formula I below, or an agronomically acceptable carboxy salt thereof (where Y in the formula is hydrogen) or an agronomically acceptable acid addition salt thereof.

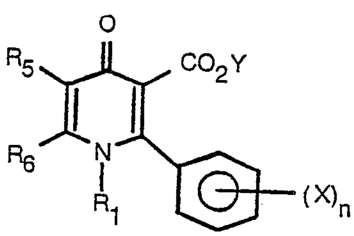

(I)

wherein $R_1$ is ($C_1$—$C_6$)alkyl or ($C_2$—$C_6$)alkenyl, each optionally substituted with a group selected from hydroxy, carboxy, carboxy salt, phenyl and phenyl substituted with up to two of the same or different substituents selected from halogen, methyl, ethyl, methoxy, ethoxy, trifluoromethyl, nitro and cyano;
$R_5$ is hydrogen, ($C_1$—$C_6$)alkyl or halogen;
$R_6$ is hydrogen or ($C_1$—$C_6$)alkyl;
X is halogen, trihalomethyl, ($C_1$—$C_6$)alkyl, nitro, cyano or ($C_1$—$C_6$)alkoxy;
n is 0 or an integer of 1—3; and
Y is hydrogen or ($C_1$—$C_6$)alkyl.

2. A composition as claimed in Claim 1, which contains at least one compound of the formula:

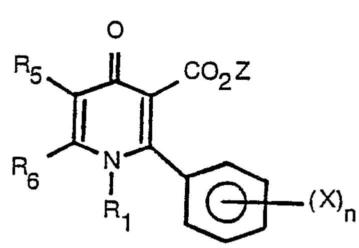

(II)

wherein $R_1$, $R_5$, $R_6$, X and n are as defined in Claim 1 and Z is hydrogen or an alkali metal and the agronomically acceptable acid addition salts thereof.

3. A composition as claimed in Claim 1, which contains at least one compound or acid addition salt as defined in Claim 2 wherein

$R_1$ is ($C_1$—$C_6$) alkyl or allyl;
$R_5$ is hydrogen, bromine or ($C_1$—$C_3$) alkyl;
$R_6$ is ($C_1$—$C_6$) alkyl;
Z is hydrogen, sodium or potassium,
X is halogen; and
n is 0, 1 or 2

4. A composition as claimed in Claim 1, which contains at least one compound or acid addition salt as defined in Claim 3, wherein

$R_1$ is ($C_1$—$C_3$) alkyl;
$R_5$ is hydrogen;
$R_6$ is ($C_1$—$C_3$) alkyl;
X is chlorine or fluorine; and
n is 0, 1 or 2.

5. A composition as claimed in Claim 1, which contains at least one compound or acid addition salt as defined in Claim 4, wherein $R_1$ is methyl or ethyl; $R_6$ is methyl; and Z is sodium or potassium.

6. A composition as claimed in Claim 1, which contains at least one compound or acid addition salt as defined in Claim 5, wherein $R_1$ is ethyl, $R_6$ is methyl and $(X)_n$ is hydrogen, 4-chloro, 3-chloro, 3,4-dichloro, or 3-fluoro.

7. A composition as claimed in Claim 1, which contains at least one compound or acid addition salt as defined in Claim 6 wherein $(X)_n$ is 4-chloro and Z is potassium.

8. A composition as claimed in Claim 1, which contains at least one compound as defined in Claim 6, wherein $R_1$ is methyl, $R_6$ is methyl and $(X)_n$ is 3-chloro, 4-chloro, or 3,4-dichloro.

9. A composition as claimed in any one of Claims 1—8 which contains water as carrier and which also contains a surface active agent.

10. A method of regulating the growth of a monocotyledonous crop plant which comprises applying to the growing plant, or to seeds thereof, a growth regulating amount of a composition as claimed in any one of Claims 1—9.

11. A method as claimed in claim 10 as applied to the production of hybrid seed by using at least one growth regulating compound which induces male sterility without destroying female fertility.

12. A method as claimed in Claim 11 wherein the plant is maize, barley, wheat, sorghum or a forage crop plant.

**Revendications pour les Etats contractants: BE CH DE FR GB IT LI LU NL SE**

1. Un composé de formule I ci-dessous, ou un de ses sels carboxyliques acceptables en agronomie (lorsque Y dans la formule est un hydrogène) ou un de ses sels d'addition d'acides acceptables en agronomie

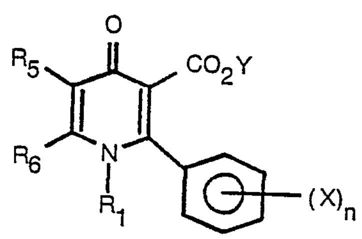

(I)

dans laquelle $R_1$ est un alcoyle ($C_1$—$C_6$) ou un alcényle ($C_2$—$C_6$), chacun d'eux étant éventuellement substitué par un radical choisi parmi hydroxy, carboxy, sel carboxylique phényle et phényle substitué par jusqu'à deux substituants semblables ou différents choisis parmi halogène, méthyle, éthyle, méthoxy, éthoxy, trifluorométhyle, nitro et cyano;

$R_5$ est un hydrogène, un alcoyle ($C_1$—$C_6$) ou un halogène;
$R_6$ est un hydrogène ou un alcoyle ($C_1$—$C_6$);
X est un halogène, un trihalogénométhyle, un alcoyle ($C_1$—$C_6$), un nitro, un cyano ou un alcoxy ($C_1$—$C_6$);
n est 0 ou un entier de 1 à 3; et
Y est un hydrogène ou un alcoyle ($C_1$—$C_6$).

2. Un composé comme revendiqué dans la revendication 1, qui a pour formule

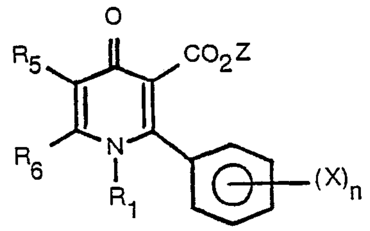

(II)

dans laquelle $R_1$, $R_5$, $R_6$, X et n sont comme défini dans la revendication 1 et Z est un hydrogène ou un métal alcalin et leurs sels d'addition d'acides acceptables en agronomie.

3. Un composé ou sel d'addition d'acide comme revendiqué dans la revendication 2, où
$R_1$ est un alcoyle ($C_1$—$C_6$) ou un allyle;
$R_5$ est un hydrogène, un brome ou un alcoyle ($C_1$—$C_3$);
$R_6$ est un alcoyle ($C_1$—$C_6$);
Z est un hydrogène, un sodium ou un potassium;
X est un halogène; et
n est 0, 1 ou 2.

4. Un composé ou sel d'addition d'acide comme revendiqué dans la revendication 3, où
$R_1$ est un alcoyle ($C_1$—$C_3$);
$R_5$ est un hydrogène;
$R_6$ est un alcoyle ($C_1$—$C_3$);
X est un chlore ou un fluor; et
n est 0, 1 ou 2.

5. Un composé ou sel d'addition d'acide comme revendiqué dans la revendication 4, où $R_1$ est un méthyle ou un éthyle; $R_6$ est un méthyle et Z est un sodium ou un potassium.

6. Un composé ou sel d'addition d'acide comme revendiqué dans la revendication 5, où $R_1$ est un éthyle, $R_6$ est un méthyle et $(X)_n$ est un hydrogène, un 4-chloro, un 3-chloro, un 3,4-dichloro ou un 3-fluoro.

7. Un composé ou sel d'addition d'acide comme revendiqué dans la revendication 6, où $(X)_n$ est un 4-chloro et Z est le potassium.

8. Un composé comme revendiqué dans la revendication 5, où $R_1$ est un méthyle, $R_6$ est un méthyle et $(X)_n$ est un 3-chloro, 4-chloro ou 3,4-dichloro.

9. Une composition de régulation de la croissance contenant comme ingrédient actif un ou plusieurs composés comme revendiqué dans toute revendication précédente et un de leurs véhicules acceptables en agronomie.

10. Une composition comme revendiquée dans la revendication 9, dans laquelle le véhicule est l'eau et un agent tensio-actif est également présent.

11. Un procédé de régulation de la croissance d'une plante cultivée monocotylédone qui comprend l'application à la plante en croissance ou à ses semences, d'une quantité provoquant la régulation de la croissance d'un ou plusieurs composés comme revendiqué dans l'une quelconque des revendications 1 à 8.

12. Un procédé comme revendiqué dans la revendication 11, appliqué à la production de semences hybrides par emploi d'au moins un composé de régulation de la croissance qui provoque la stérilité mâle sans détruire la fertilité femelle.

13. Un procédé comme revendiqué dans la revendication 12, dans lequel la plante est le maïs, l'orge, le blé, le sorgho ou une plante fourragère cultivée.

**Revendications pour l'Etat contractant: AT**

1. Une composition de régulation de la croissance des plantes contenant, comme ingrédient actif, au moins un composé qui provoque la stérilité mâle chez les plantes monocotylédones et un véhicule ou diluant acceptables en agronomie de celui-ci, caractérisée en ce qu'elle contient au moins un composé de formule I ci-dessous, ou un sel carboxylique acceptable en agronomie (lorsque Y dans la formule est un hydrogène) ou un sel d'addition d'acide acceptable en agronomie de celui-ci,

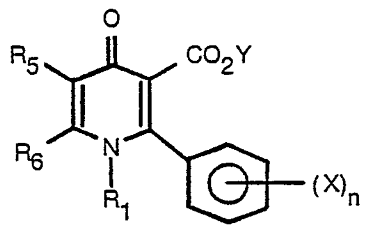

(I)

dans laquelle $R_1$ est un alcoyle ($C_1$—$C_6$) ou un alcényle ($C_2$—$C_6$), chacun d'eux étant éventuellement substitué par un radical choisi parmi un hydroxy, un carboxy, un sel carboxylique, un phényle et un phényle substitué par jusqu'à deux substituants semblables ou différents choisis parmi halogène, méthyle, éthyle, méthoxy, éthoxy, trifluorométhyle, nitro et cyano;

$R_5$ est un hydrogène, un alcoyle ($C_1$—$C_6$) ou un halogène;

$R_6$ est un hydrogène ou un alcoyle ($C_1$—$C_6$);

X est un halogène, un trihalogénométhyle, un alcoyle ($C_1$—$C_6$), un nitro, un cyano ou un alcoxy ($C_1$—$C_6$);

n est 0 ou un entier de 1 à 3; et

Y est un hydrogène ou un alcoyle ($C_1$—$C_6$).

2. Une composition comme revendiquée dans la revendication 1, qui contient au moins un composé de formule

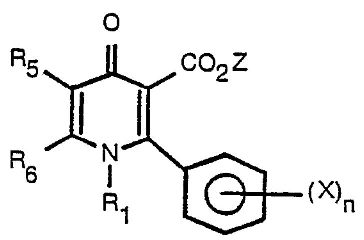

(II)

dans laquelle $R_1$, $R_5$, $R_6$, X et n sont comme défini dans la revendication 1 et Z est un hydrogène ou un métal alcalin et leurs sels d'addition d'acides acceptables en agronomie.

3. Une composition comme revendiquée dans la revendication 1, qui contient au moins un composé ou sel d'addition d'acide comme défini dans la revendication 2, où

$R_1$ est un alcoyle ($C_1$—$C_6$) ou un allyle;

$R_5$ est un hydrogène, un brome ou un alcoyle ($C_1$—$C_3$);

$R_6$ est un alcoyle ($C_1$—$C_6$);

Z est un hydrogène, un sodium ou un potassium;

X est un halogène; et

n est 0, 1 ou 2.

4. Une composition comme revendiquée dans la revendication 1, qui contient au moins un composé ou sel d'addition d'acide comme défini dans la revendication 3, où

$R_1$ est un alcoyle ($C_1$—$C_3$);

$R_5$ est un hydrogène;

$R_6$ est un alcoyle ($C_1$—$C_3$);

X est un chlore ou un fluor; et

n est 0, 1 ou 2.

5. Une composition comme revendiquée dans la revendication 1, qui contient au moins un composé ou sel d'addition d'acide comme défini dans la revendication 4, où $R_1$ est un méthyle ou un éthyle; $R_6$ est un méthyle; et Z est le sodium ou le potassium.

6. Une composition comme revendiquée dans la revendication 1, qui contient au moins un composé ou sel d'addition d'acide comme défini dans la revendication 5, où $R_1$ est un éthyle, $R_6$ est un méthyle et $(X)_n$ est un hydrogène, un 4-chloro, un 3-chloro, un 3,4-dichloro ou un 3-fluoro.

7. Une composition comme revendiquée dans la revendication 1, qui contient au moins un composé ou sel d'addition d'acide comme défini dans la revendication 6, où $(X)_n$ est un 4-chloro et Z est le potassium.

8. Une composition comme revendiquée dans la revendication 1, qui contient au moins un composé comme défini dans la revendication 6, où $R_1$ est un méthyle, $R_6$ est un méthyle et $(X)_n$ est un 3-chloro, 4-chloro, ou 3,4-dichloro.

9. Une composition comme revendiquée dans l'une quelconque des revendications 1 à 8, qui contient de l'eau comme véhicule et qui contient aussi un agent tensio-actif.

10. Un procédé de régulation de la croissance d'une plante cultivée monocotylédone qui comprend l'application à la plante en croissance ou à ses semences d'une quantité provoquant la

régulation de la croissance d'une composition comme revendiquée dans l'une quelconque des revendications 1 à 9.

11. Un procédé comme revendiqué dans la revendication 10, appliqué à la production de semences hybrides par emploi d'au moins un composé de régulation de la croissance qui provoque la stérilité mâle sans détruire la fertilité femelle.

12. Un procédé comme revendiqué dans la revendication 11, dans lequel la plante est le maïs, l'orge, le blé, le sorgho ou une plante fouragère cultivée.

**Patentansprüche für die Vertragsstaaten: BE CH DE FR GB IT LI LU NL SE**

1. Verbindung der nachfolgenden Formel I oder ein für landwirtschaftliche Zwecke verträgliches Carboxysalz davon (wobei Y in der Formel für Wasserstoff steht) oder ein für landwirtschaftliche Zwecke verträgliches Säureadditionssalz davon

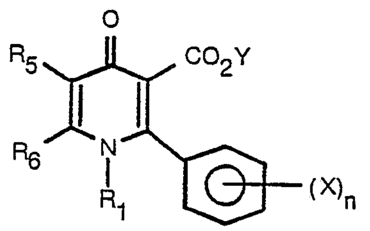

(I)

worin $R_1$ für $(C_1—C_6)$Alkyl oder $(C_2—C_6)$Alkenyl steht, jeweils gegebenenfalls substituiert mit einer Gruppe, ausgewählt aus Hydroxy, Carboxy, Carboxysalz, Phenyl sowie Phenyl, substituiert mit bis zu zwei gleichen oder verschieden Substituenten ausgewählt aus Halogen, Methyl, Ethyl, Methoxy, Ethoxy, Trifluormethyl, Nitro und Cyano, $R_5$ Wasserstoff, $(C_1—C_6)$Alkyl oder Halogen ist, $R_6$ Wasserstoff oder $(C_1—C_6)$Alkyl bedeutet, X Halogen, Trihalogenmethyl, $(C_1—C_6)$Alkyl, Nitro, Cyano oder $(C_1—C_6)$Alkoxy bedeutet, n 0 oder eine ganze Zahl von 1 bis 3 ist und Y Wasserstoff oder $(C_1—C_6)$Alkyl versinnbildlicht.

2. Verbindung nach Anspruch 1, dadurch gekennzeichnet, daß sie der Formel

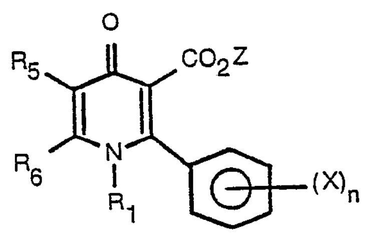

(II)

entspricht, worin $R_1$, $R_5$, $R_6$, X und n die in Anspruch 1 angegebenen Bedeutungen besitzen und Z Wasserstoff oder ein Alkalimetall ist, sowie die für landwirtschftliche Zwecke verträglichen Säureadditionssalze davon.

3. Verbindung oder Säureadditionssalz gemäß Anspruch 2, wobei $R_1$ für $(C_1—C_6)$Alkyl oder Allyl steht, $R_5$ Wasserstoff, Brom oder $(C_1—C_3)$Alkyl ist, $R_6$ $(C_1—C_6)$Alkyl bedeutet, Z Wasserstoff, Natrium oder Kalium darstellt, X Halogen ist und n 0, 1 oder 2 ist.

4. Verbindung oder Säureadditionssalze nach Anspruch 3, dadurch gekennzeichnet, daß $R_1$ für $(C_1—C_3)$Alkyl steht, $R_5$ Wasserstoff bedeutet, $R_6$ $(C_1—C_3)$Alkyl ist, X Chlor oder Fluor bedeutet und n 0, 1 oder 2 ist.

5. Verbindung oder Säureadditionssalz nach Anspruch 4, dadurch gekennzeichnet, daß $R_1$ für Methyl oder Ethyl steht, $R_6$ Methyl ist und Z Natrium oder Kalium bedeutet.

6. Verbindung oder Säureadditionssalz gemäß Anspruch 5, dadurch gekennzeichnet, daß $R_1$ für Ethyl steht, $R_6$ Methyl ist und $(X)_n$ Wasserstoff, 4-Chlor, 3-Chlor, 3,4-Dichlor oder 3-Fluor bedeutet.

7. Verbindung oder Säureadditionssalz gemäß Anspruch 6, dadurch gekennzeichnet, daß $(X)_n$ für 4-Chlor steht und Z Kalium bedeutet.

8. Verbindung nach Anspruch 5, dadurch gekennzeichnet, daß $R_1$ Methyl ist, $R_6$ Methyl darstellt und $(X)_n$ 3-Chlor, 4-Chlor oder 3,4-Dichlor bedeutet.

9. Zusammensetzung zur Wachstumsregulierung, enthaltend als aktiven Bestandteil eine oder mehrere Verbindung gemäß einem der vorhergehenden Ansprüche und ein landwirtschaftlich annehmbaren Träger dafür.

10. Zusammensetzung nach Anspruch 9, dadurch gekennzeichnet, daß der Träger Wasser und auch ein oberflächenaktives Mittel vorhanden ist.

11. Verfahren zur Regulierung des Wachstums einer einkeimblättrigen Nutzpflanze, dadurch gekennzeichnet, daß man auf die wachsende Pflanze oder die Samen davon eine das Wachstum regulierende Menge von einer oder mehreren Verbindungen gemäß einem der Ansprüche 1 bis 8 aufbringt.

12. Verfahren nach Anspruch 11, angewandt auf die Erzeugung von Hybridsaatgut durch Anwendung von zumindest einer wachtstumsregulierenden Verbindung, welche männliche Sterilität ohne Zerstörung der weiblichen Fruchtbarkeit induziert.

13. Verfahren nach Anspruch 12, dadurch gekennzeichnet, daß die eingesetzten Pflanzen Mai, Gerste, Weizen, Sorghum oder eine Futternutzpflanze ist.

**Patentansprüche für den Vertraagsstaat: AT**

1. Pflanzenwachstumsregulierendes Mittel, das als Wirkstoff wenigstens eine Verbindung, die eine männliche Sterilität in einkeimblättrigen Pflanzen erzeugt, sowie einen für landwirtschaftliche Zwecke verträglichen Träger oder ein für landwirtschaftliche Zwecke verträgliches Verdünnungsmittel dafür enthält, dadurch gekennzeichnet, daß es wenigstens eine Verbindung der folgenden Formel I oder ein für landwirtschaftliche Zwecke verträgliches Carboxysalz davon (wobei Y in der Formel für Wasserstoff steht) oder ein für landwirtschaftliche Zwecke verträgliches Säureadditionssalz davon enthält

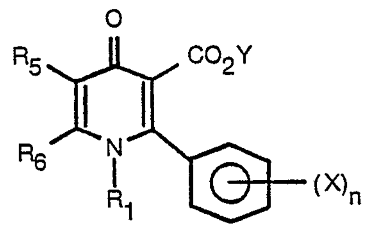

(I)

worin $R_1$ für $(C_1—C_6)$Alkyl oder $(C_2—C_6)$Alkenyl steht, jeweils gegebenenfalls substituiert mit einer Gruppe, ausgewählt aus Hydroxy, Carboxy, Carboxysalz, Phenyl und Phenyl, substituiert mit bis zu zwei gleichen oder verschiedenen Substituenten, ausgewählt aus Halogen, Methyl, Ethyl, Methoxy, Ethoxy, Trifluormethyl, Nitro und Cyano,

$R_5$ Wasserstoff, $(C_1—C_6)$Alkyl oder Halogen bedeutet,

$R_6$ Wasserstoff oder $(C_1—C_6)$Alkyl ist,

X Halogen, Trihalogenmethyl, $(C_1—C_6)$Alkyl, Nitro, Cyano oder $(C_1—C_6)$Alkoxy bedeutet,

n 0 oder eine ganze Zahl von 1 bis 3 ist und

Y Wasserstoff oder $(C_1—C_6)$Alkyl ist.

2. Mittel nach Anspruch 1, dadurch gekennzeichnet, daß es wenigstens eine Verbindung der Formel

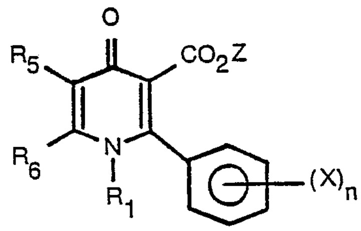

(II)

worin $R_1$, $R_5$, $R_6$, X und n die in Anspruch 1 angegebene Bedeutung besitzen und Z Wasserstoff oder ein Alkalimetall ist, oder ein für landwirtschaftliche Zwecke verträgliche Säureadditionssalze davon enthält.

3. Mittel nach Anspruch 1, dadurch gekennzeichnet, daß es wenigstens eine Verbindung oder ein Säureadditionssalz gemäß Anspruch 2 enthält, wobei

$R_1$ für $(C_1—C_6)$Alkyl oder Allyl steht,

$R_5$ Wasserstoff, Brom oder $(C_1—C_3)$Alkyl ist,

$R_6$ $(C_1—C_6)$Alkyl ist,

Z Wasserstoff, Natrium oder Kalium bedeutet,

X Halogen ist und

n 0, 1 oder 2 ist.

4. Mittel nach Anspruch 1, dadurch gekennzeichnet, daß es wenigstens eine Verbindung oder ein Säureadditionssalz gemäß Anspruch 3 enthält, wobei

$R_1$ für $(C_1—C_3)$Alkyl steht,

$R_5$ Wasserstoff ist,

$R_6$ $(C_1—C_3)$Alkyl ist,

X Chlor oder Fluor ist und
n 0, 1 oder 2 bedeutet.

5. Mittel nach Anspruch 1, dadurch gekennzeichnet, daß es wenigstens eine Verbindung oder ein Säureadditionssalz gemäß Anspruch 4 enthält, wobei $R_1$ für Methyl oder Ethyl steht, $R_6$ Methyl bedeutet und Z Natrium oder Kalium ist.

6. Mittel nach Anspruch 1, dadurch gekennzeichnet, daß es wenigstens eine Verbindung oder ein Säureadditionssalz gemäß Anspruch 5 enthält, wobei $R_1$ für Ethyl steht, $R_6$ Methyl ist und $(X)_n$ Wasserstoff, 4-Chlor, 3-Chlor, 3,4-Dichlor oder 3-Fluor bedeutet.

7. Mittel nach Anspruch 1, dadurch gekennzeichnet, daß es wenigstens eine Verbindung oder ein Säureadditionssalz gemäß Anspruch 6 enthält, wobei $(X)_n$ für 4-Chlor steht und Z Kalium ist.

8. Mittel nach Anspruch 1, dadurch gekennzeichnet, daß est wenigstens eine Verbindung gemäß Anspruch 6 enthält, wobei $R_1$ für Methyl steht, $R_6$ Methyl ist und $(X)_n$ 3-Chlor, 4-Chlor oder 3,4-Dichlor bedeutet.

9. Mittel nach einem der Ansprüche 1 bis 8, dadurch gekennzeichnet, daß es Wasser als Träger enthält und außerdem ein grenzflächenaktives Mittel enthält.

10. Verfahren zur Regulierung des Wachstums einer einkeimblättrigen Nutzpflanze, dadurch gekennzeichnet, daß auf die wachsende Pflanze oder auf ihr Saatgut eine wachstumsregulierende Menge eines Mittels gemäß einem der Ansprüche 1 bis 9 aufgebracht wird.

11. Verfahren nach Anspruch 10, dadurch gekennzeichnet, daß es auf die Erzeugung eines Hybridsaatguts durch Verwendung wenigstens einer wachstumsregulierenden Menge angewendet wird, die eine männliche Sterilität ohne Zerstörung der weiblichen Fertilität induziert.

12. Verfahren nach Anspruch 11, dadurch gekennzeichnet, daß die eingesetzten Pflanzen aus Mais, Gerste, Weizen, Sorghum oder einer Futternutzpflanze besteht.